# EUROPÄISCHE PATENTANMELDUNG

(11) **EP 2 546 229 A1**
(43) Veröffentlichungstag der Anmeldung: **16.01.2013**
(21) Anmeldenummer: 11005807.0
(22) Anmeldetag: 15.07.2011
(51) Int. Cl.: C07C 209/68, C07C 213/08

(54) **Verfahren zur Synthese von Aminobiphenylen**

(71) Anmelder: Friedrich-Alexander-Universität Erlangen-Nürnberg, 91054 Erlangen (DE)
(72) Erfinder: Heinrich, Markus, 91094 Langensendelbach (DE); Pratsch, Gerald, 85356 Freising (DE)
(74) Vertreter: Forstmeyer, Dietmar

(57) **Zusammenfassung**

Die vorliegende Erfindung beschreibt ein Verfahren zur Synthese von 2-Aminobiphenylen sowie Derivaten davon. Dieses Verfahren ist kostengünstig durchführbar und beruht auf selektiven Umsetzungen. Funktionalisierte Biphenylverbindungen sind insbesondere als Pharmazeutika und Pflanzenschutzmittel sowie als Vorstufen solcher Wirkstoffe von großem Interesse. Das Verfahren zur Herstellung einer Verbindungen der Formel 3 ist dadurch gekennzeichnet, dass eine Verbindung der Formel 1 mit einer Verbindung der Formel 2 umgesetzt wird.

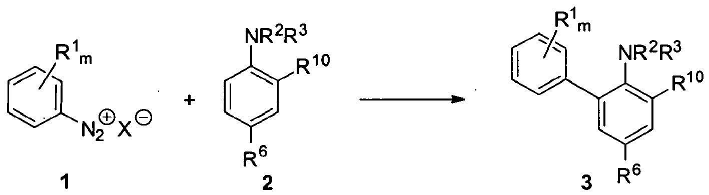

## Beschreibung

Die vorliegende Erfindung beschreibt ein Verfahren zur Synthese von 2-Aminobiphenylen sowie Derivaten davon. Dieses Verfahren ist kostengünstig durchführbar und beruht auf selektiven Umsetzungen. Funktionalisierte Biphenylverbindungen sind insbesondere als Pharmazeutika und Pflanzenschutzmittel sowie als Vorstufen solcher Wirkstoffe von großem Interesse.

Eine breite Palette metallorganischer Methoden steht heute zur milden und effizienten Synthese von Biarylverbindungen zur Verfügung. Aktuelle Übersichtsartikel zu dieser Thematik wurden von Beller, Bolm, de Meijere und Diederich verfasst. [M. Beller, C. Bolm, Transition Metals for Organic Synthesis, 2nd ed., Wiley-VCH, Weinheim, 2004**;** A. de Meijere, F. Diederich, Metal-Catalyzed Cross-Coupling Reactions, 2nd ed., Wiley-VCH, Weinheim, 2004**;** L. Ackermann, Modern Arylation Methods, 1st ed., Wiley-VCH, Weinheim, 2009**.**]

Kürzlich erschienene Artikel, in denen neueste Entwicklungen beschrieben werden, wurden von folgenden Arbeitsgruppen publiziert: [T. Dohi, M. Ito, K. Morimoto, M. Iwata, Y. Kita, Angew. Chem. 2008, 120, 1321-1324, Angew. Chem. Int. Ed. 2008, 47, 1301-1304; M. Amatore, C. Gosmini, Angew. Chem. 2008, 120, 2119-2122, Angew. Chem. Int. Ed. 2008, 47, 2089-2092; L. J. Gooßen, N. Rodriguez, K. Gooßen, Angew. Chem. 2008, 120, 3144-3164, Angew. Chem. Int. Ed. 2008, 47, 3100-3120.]

Die bekannten metallorganischen Methoden sind allerdings auch mit Nachteilen behaftet. Ihre Attraktivität wird beispielsweise durch hohe Kosten der Ausgangsmaterialien, insbesondere bei Palladium-katalysierten Umsetzungen, oder mangelnde Umweltverträglichkeit, wie im Falle des Nickels, gemindert. Katalytische Verfahren unter Verwendung von Cobalt- und Eisenverbindungen sind bisher nur eingeschränkt anwendbar.

Einfachere Ausgangsmaterialien können eingesetzt werden, wenn die Biarylkupplung über eine CH-Bindungsaktivierung am Aromaten erfolgt. G. Dyker, Handbook of C-H Transformations, Wiley-VCH, Weinheim, 2005**;** L. Ackermann, Top. Organomet. Chem. 2007, 24, 35-60; T. Vogler, A. Studer, Org. Lett. 2008, 10, 129-131; L. Ackermann, R. Vicente, A. Althammer, Org. Lett. 2008, 10, 2299-2302. Trotz zahlreicher aktueller Arbeiten auf diesem Forschungsgebiet ist das verwendbare Substratspektrum bisher noch sehr begrenzt.

Verglichen mit der Vielfalt an metallorganischen Transformationen, die im Wesentlichen in den letzten zwei Jahrzehnten entwickelt wurden, kommen Additionsreaktionen von Arylradikalen an aromatische Substrate aktuell nur selten zum Einsatz.

Dabei liegen die Pionierarbeiten von Pschorr, Gomberg und Bachmann auf dem Gebiet der radikalischen Biarylsynthese, in der traditionell Aryldiazoniumsalze als Radikalvorläufer eingesetzt werden, bereits lange zurück. [M. Gomberg, W. E. Bachmann, J. Am. Chem. Soc. 1924, 46, 2339-2343, R. Pschorr, Chem. Ber. 1896, 29, 496-501] Ein grundlegender Nachteil der intermolekularen Reaktionsführung besteht allerdings darin, dass die Addition von Arylradikalen an gängige Substrate wie substituierte Benzole meist nur langsam erfolgt, was in der Folge Nebenreaktionen begünstigt. [J. C. Scaiano, L. C. Stewart, J. Am. Chem. Soc. 1983, 105, 3609-3614] Der Erfolg radikalischer Biarylsynthesen ist deshalb häufig an spezielle Bedingungen geknüpft, wobei das Substrat als Lösungsmittel eingesetzt [A. Nunez, A. Sanchez, C. Burgos, J. Alvarez-Builla, Tetrahedron 2004, 60, 6217-6224, P. T. F. McLoughlin, M. A. Clyne, F. Aldabbagh, Tetrahedron 2004, 60, 8065-8071] oder die Reaktion intramolekular durchführt wird [M. L. Bennasar, T. Roca, F. Ferrando, Tetrahedron Lett. 2004, 45, 5605-5609]. Eine Verbesserung der klassischen Gomberg-Bachmann Reaktion konnte auch durch eine Reaktionsführung unter Phasentransferbedingungen erzielt werden. [J. R. Beadle, S. H. Korzeniowski, D. E. Rosenberg, B. J. Garcia-Slanga, G. W. Gokel, J. Org. Chem. 1984, 49, 1594-1603]

Aus kürzlich erschienen Übersichtsartikeln zur radikalischen Biarylsynthese geht hervor, dass in der aktuellen Forschung Aryldiazoniumsalze zunehmend durch Arylchloride, -bromide und iodide als Radikalvorläufer ersetzt werden. [A. Studer, M. Brossart in Radicals in Organic Synthesis, Eds. P. Renaud, M. P. Sibi, 1st ed., Wiley-VCH, Weinheim, 2001, Vol. 2, 62-80; W. R. Bowman, J. M. D. Storey, Chem. Soc. Rev. 2007, 36, 1803-1822; J. Fossey, D. Lefort, J. Sorba, Free Radicals in Organic Chemistry, Wiley, Chichester, 1995, 167-180.] Allerdings müssen zur Generierung von Arylradikalen aus Arylhalogeniden meist toxische Organozinn- oder teure Organosiliciumverbindungen eingesetzt werden.

Basierend auf der grundsätzlichen Attraktivität von Aryldiazoniumsalzen als Vorläufer von Arylradikalen [C. Galli, Chem. Rev. 1988, 88, 765-792], die vor allem durch die geringe Toxizität und einfache Zugänglichkeit aus Anilinverbindungen begründet ist, besteht eine wesentliche Herausforderung in der Erweiterung des bekannten Substratspektrums hinsichtlich der Synthese von Biarylverbindungen.

In diesem Zusammenhang stellen vor allem Aniline eine bedeutende Substratgruppe dar. Einzelne Beispiele für Additionsreaktionen von Arylradikalen an Anilinderivate sind zwar seit langem bekannt, doch hat diese Synthesemethode für Biarylamine bisher keine Bedeutung erlangt. Allan und Muzik [Chem. Abstr. 1953, 8705] berichten beispielsweise über die Umsetzung des Diazoniumsalzes vonpara-Nitroanilin mit Benzidin sowie *N,N,N;N'-*Tetramethylbenzidin. Dabei geht nur das Tetramethylderivat die radikalische Biarylkupplung ein, während das unsubstituierte Benzidin nach einem nichtradikalischen Mechanismus zum entsprechenden Triazen reagiert. Ein der Methylsubstitution ähnlicher Effekt kann erzielt werden, wenn Anilinverbindungen nicht als freie Basen, sondern als Aniliniumsalze mit Arylradikalen zur Reaktion gebracht werden. Erste Arbeiten zur radikalischen Arylierung von protoniertem 1,4-Phenyldiamin wurden ebenfalls von Allan und Muzik beschrieben [Z. J. Allan, F. Muzik, Chem. Listy 1954, 48,52]. Neuere Untersuchungen unter verbesserten Reaktionsbedingungen führten zu einer wesentlichen Erweiterung des Substratspektrums, die Anilinverbindungen mussten aber stets noch in protonierter Form in der Reaktionslösung vorliegen.[Angew. Chem. Int. Ed. 2008, 47, 9130-9133, WO2010000856, WO2010037531] Begründet durch die Protonierung und der daraus resultierenden, verlangsamten Addition des Arylradikals an das Aniliniumsalz, konnte ein großer Teil der Anilinverbindungen nur in mäßigen Ausbeuten zu Biarylaminen umgesetzt werden. Wären Aniline dagegen unprotoniert mit Arylradikalen umsetzbar, so sind deutlich höhere Ausbeuten und bessere Selektivitäten zu erwarten. Andererseits ist speziell im Zusammenhang mit Aryldiazoniumsalzen die Protonierung (oder Dialkylsubstitution) der Aminogruppe bisher essentiell, da nur auf diese Weise eine wirkungsvolle Zurückdrängung der sonst vorherrschenden Reaktionswege von Triazenbildung und Azokupplung gelingt.

Funktionalisierte Biphenylverbindungen sind insbesondere als Pharmazeutika und Pflanzenschutzmittel sowie als Vorstufen solcher Wirkstoffe von großem Interesse.

Der vorliegenden Erfindung lag die Aufgabe zu Grunde, ein Verfahren zur Herstellung von 2-Aminobiphenylen und Derivaten bereitzustellen, das vorzugsweise auf der radikalischen Arylierung von unprotonieren Anilinverbindungen beruht. Aus Kostengründen und bedingt durch ihre einfache Zugänglichkeit sollten Aryldiazoniumsalze als Arylradikalvorläufer eingesetzt werden.

Die Aufgabe wird durch die im Folgenden näher beschriebenen Verfahren zur Herstellung von 2-Aminobiphenylen gelöst.

Ausführungsfonnen der Erfindung sind:

Verfahren zur Herstellung einer Verbindung der Formel 3 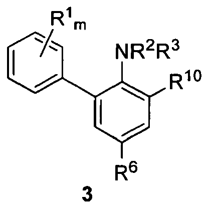 wobei eine Verbindung der Formel 1 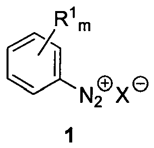 mit einer Verbindung der Formel 2 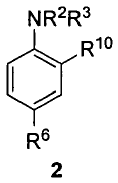 umgesetzt wird, wobei
- m: für 0, 1, 2, 3, 4 oder 5 steht;
- jedes R¹: jeweils unabhängig für Halogen, Alkyl, Haloalkyl, Hydroxy, Hydroxyalkyl, Alkoxy, Haloalkoxy, Alkylthio, Cycloalkyl, Haloalkylthio, Alkenyl, Alkinyl, Amino, Nitro, Cyano, -SO₃R⁵, -SO₂NH₂, -SO₂NHR⁴, -SO₂NR⁴R⁵, -COOR⁴, -CONHR⁴, -CONR⁴R⁵, -COR⁴, -OCOR⁴,-NR⁴R⁵, -NR⁴COR⁵, -NR⁴SO₂R⁵, Alkylcarbonyl, Haloalkylcarbonyl, Alkenylcarbonyl, Alkoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Heteroarylalkyl, Arylalkoxycarbonyl, Arylalkylimino oder Heteroaryl steht;
- X⁻: fiir Halogenid, Hydrogensulfat, Sulfat, Tetrafluoroborat, Acetat, Trifluoracetat, Hexafluorophosphat, Hexafluoroantimonat, das Anion eines aromatischen 1,2-Dicarbonsäureimids oder das Anion eines aromatischen 1,2-Disulfonsäureimids steht;
- R² und R³: jeweils unabhängig für Wasserstoff, Alkyl, Hydroxyalkyl, Aminoalkyl, Cycloalkyl, Haloalkyl, -(CH₂)ₙ-OR⁴, -(CH₂)ₙ-NR⁴R⁵, -(CH₂)ₙ-NR⁴COR⁵, -(CH₂)ⁿ-NR⁴COOR⁵, -(CH₂)ₙ-COOR⁴, -(CH₂)ₙ-CONHR⁴, -(CH₂)ₙ-CONR⁴R⁵, -(CH₂)ₙ-SO₃R⁴, -(CH₂)ₙ-CN, Arylalkyl, Heteroarylalkyl, Aryl oder Heteroaryl steht, oder ein R² und ein R³ zusammen einen Alkylidenrest bilden, oder ein R² und ein R³ zusammen ein nichtaromatisches Ringsystem bilden, welches 4, 5, 6, oder 7 Ringatome enthält, ausgewählt aus O, S, N und Kohlenstoffatomen, vorzugsweise mit Kohlenstoffatomen, oder ein R² und ein R¹⁰ zusammen ein nichtaromatisches Ringsystem bilden, welches 4, 5, 6, oder 7 Ringatome enthält, ausgewählt aus O, S, N und Kohlenstoffatomen, vorzugsweise mit Kohlenstoffatomen, oder ein R³ und ein R¹⁰ zusammen ein nichtaromatisches Ringsystem bilden, welches 4, 5, 6, oder 7 Ringatome enthält, ausgewählt aus O, S, N und Kohlenstoffatomen, vorzugsweise mit Kohlenstoffatomen;
- n: jeweils unabhängig für 1, 2, 3, 4, 5, 6, 7, 8, 9 oder 10 steht;
- R⁴: jeweils unabhängig für Wasserstoff, Alkyl, Cycloalkyl, Haloalkyl, Arylalkyl, Heteroarylalkyl, Aryl oder Heteroaryl steht;
- R⁵: jeweils unabhängig für Wasserstoff, Alkyl, Cycloalkyl, Haloalkyl, Arylalkyl, Heteroarylalkyl, Aryl oder Heteroaryl steht;
- R⁶: jeweils unabhängig für Wasserstoff, Halogen, Alkyl, Alkenyl, Alkinyl, Cycloalkyl, Arylalkyl, Heteroarylalkyl, Haloalkyl, Hydroxyalkyl, Alkoxyalkyl, Aryloxyalkyl, Heteroaryloxyalkyl, Aminoalkyl, -(CH₂)ₙ-NR⁴R⁵, -COOH, -CHO, -CN, -COR⁴, Alkylcarbonyl, Haloalkylcarbonyl, Cycloalkylcarbonyl, Arylalkylcarbonyl, Alkenylcarbonyl, Arylcarbonyl, Heteroarylcarbonyl, -COOR⁴, Alkoxycarbonyl, Haloalkoxycarbonyl, Cycloalkoxycarbonyl, Arylalkoxycarbonyl, Alkenyloxycarbonyl, Aryloxycarbonyl, Heteroaryloxycarbonyl, -CONHR⁴, -CONR⁴R⁵, Amino, Nitro, -NHR⁴, -NR⁴R⁵, 1-Pyrrolidino, 1-Piperidino, 1-Morpholino, Alkylimino, Cycloalkylimino, Haloalkylimino, Arylalkylimino, -NR⁴COR⁵, NR⁴COOR⁵, -NR⁴SO₂R⁵, Hydroxy, Alkoxy, Haloalkoxy, Cycloalkoxy, Arylalkyloxy, Aryloxy, Heteroaryloxy, -OCOR⁴, Alkylcarbonyloxy, Haloalkylcarbonyloxy, Cycloalkylcarbonyloxy, Arylalkylcarbonyloxy, Arylcarbonyloxy, Heteroarylcarbonyloxy, -OCONR⁴R⁵, -O-(CH₂)ₙ-OR⁴, -O-(CH₂)ₙ-NR⁴R⁵, -O-(CH₂)ₙ-NR⁴COR⁵, -O-(CH₂)ₙ-NR⁴COOR⁵, -O-(CH₂)ₙ-COOR⁴, -O-(CH₂)ₙ-CONHR⁴, -O-(CH₂)ₙ-CONR⁴R⁵, -O-(CH₂)ₙ-SO₃R⁴, -O-(CH₂)ₙ-SO₂R⁴, -O-(CH₂)ₙ-CN, -SH, Alkylthio, Haloalkylthio, Cycloalkylthio, Arylalkylthio, Arylthio, Heteroarylthio, Alkylsulfonyl, Haloalkylsulfonyl, Cycloalkylsulfonyl, Arylalkylsulfonyl, Arylsulfonyl, Heteroarylsulfonyl, -SO₂NH₂, -SO₂NHR⁴, -SO₂NR⁴R⁵, -SO₃R⁵, Aryl oder Heteroaryl steht;
- R¹⁰: jeweils unabhängig für Wasserstoff, Halogen, Alkyl, Haloalkyl, Hydroxyalkyl, Cycloalkyl, Arylalkyl, Heteroarylalkyl, -(CH₂)_{q}-NR⁴R⁵, -(CH₂)_{q}-NR⁴COR⁵, -(CH₂)_{q}-NR⁴COOR⁵, -(CH₂)_{q}-COOR⁴, -(CH₂)_{q}-CONHR⁴, -(CH₂)_{q}-CONR⁴R⁵, -(CH₂)_{q}-SO₃R⁴, -(CH₂)_{q}-CN, Aryl oder Heteroaryl steht;
- q: jeweils unabhängig für 1, 2, 3, 4, oder 5 steht,
dadurch gekennzeichnet, dass die Umsetzung im basischen Bereich durchgeführt wird.

Im Besonderen wird ein Verfahren zur Synthese von substituierten 2-Aminobiphenylen der Struktur 3 durch Umsetzung von substituierten Aryldiazoniumsalzen der Struktur 1 mit substituierten Anilinverbindungen der Struktur 2 beschrieben.

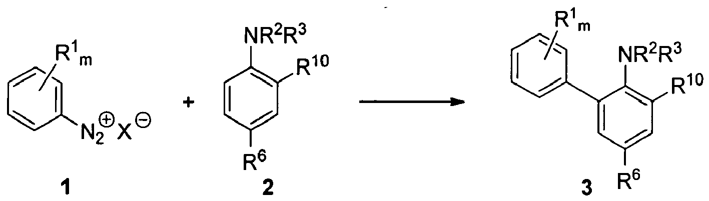

Als Zwischenprodukte entstehen dabei im basischen Bereich vorzugsweise Verbindungen der Formeln **1a** und/oder **1b** und/oder **1c:**

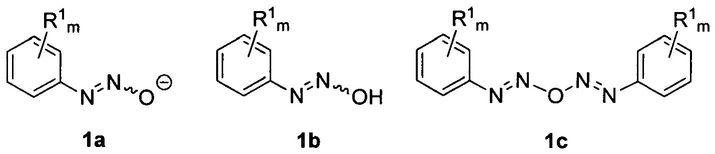

Dabei sind die Reste wie oben definiert. Das Gegenanion von Verbindung **1a** hängt dabei von der verwendeten Base ab. Bevorzugte Gegenanionen sind Na⁺ und K⁺.

Die Vorliegende Erfindung betrifft somit weiterhin ein Verfahren zur Herstellung einer Verbindung der Formel **3** 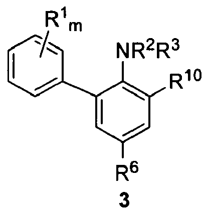 wobei in einem ersten Schritt eine Verbindung der Formel **1** 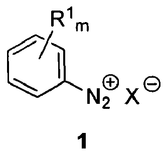 im basischen Bereich zu Verbindungen der Formeln **1a** und/oder **1b** und/oder **1c** umgesetzt wird, 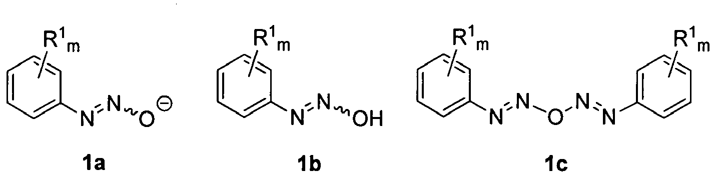 und in einem zweiten Schritt die Verbindungen **1a** und/oder **1b** und/oder **1c** im basischen Bereich mit einer Verbindung der Formel **2** 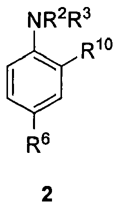 zu einer Verbindung der Formel **3** umgesetzt werden, 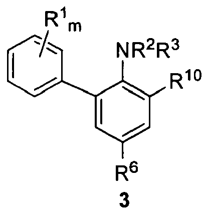 wobei alle Reste wie oben definiert sind.

Die Verbindungen der Struktur **3** können beispielsweise als Zwischenprodukte zur Herstellung biologisch aktiver Verbindungen verwendet werden.

Ein Beispiel ist das Pflanzenschutzmittel der Struktur **5,** das nach bekannten Verfahren aus einer Verbindung der Struktur **4** gewonnen werden kann (US 2010174094A1, WO 2006024388A1, US 2008269263A1, US 2010069646A1). Metallorganische Synthesen der Verbindung der Struktur **4,** die allerdings deutlich teurere Ausgangsmaterialien benötigen als das im Folgenden vorgestellte erfindungsgemäße Verfahren, sind ebenfalls kürzlich beschrieben worden. (WO2007138089A1, US2010185015A1)

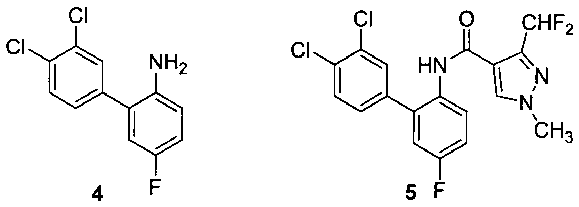

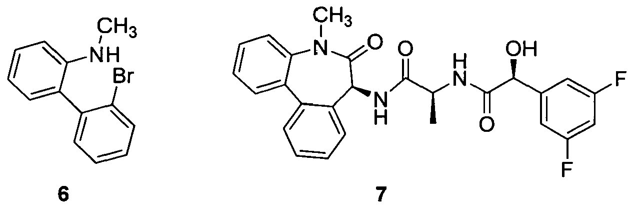

Ausgehend von der Verbindung der Struktur **6** kann z. B. ein γ-Sekretase-Inhibitor der Struktur **7** (LY411575) hergestellt werden (X. Pan, C. S. Wilcox, J. Org. Chem. 2010, 75, 6445-6451).

Des Weiteren wird ein Verfahren zur Synthese einer Verbindung der Formel **9** beschrieben, dadurch gekennzeichnet, dass in einem ersten Schritt eine Verbindung der Formel **1** mit einer Verbindung der Formel **2,** wobei R² und R³ = H, zu einer Verbindung der Formel **3,** wobei R² und R³ = H, (vorzugsweise wie oben beschrieben) umgesetzt wird und in einem weiteren Schritt die Verbindung der Formel **3** in eine Verbindung der Formel **8** überführt wird. Die Herstellung von divers funktionalisierten Verbindungen der Formel **8** gelingt unter den unten genauer ausgeführten Bedingungen zur Diazotierung aromatischer Amine.

In einem weiteren Schritt werden die Verbindungen der Formel **8** nach literaturbekannten Verfahren in Verbindungen der Formel **9** überführt. Die reduktive Deaminierung zu **9** (R¹¹ = H) kann unter verschiedensten Reaktionsbedingungen durchgeführt werden (z.B. in A. Wetzel, V. Ehrhardt, M. R. Heinrich, Angew. Chem. Int. Ed. 2008, 47, 9130-9133). Alternativ sind Halogenverbindungen, Thiole, Thioether und Nitrile der Formel **9** (R¹¹= -SH, -SAlkyl, -SHaloalkyl, -SAryl, -SHeteroaryl, Halogen, Cyano) unter den Bedingungen der Sandmeyer-Reaktion zugänglich (F. Minisci, F. Fontana, E. Vismara, Gazz. Chim. Ital. 1993, 123, 9-18). Umsetzungen von **8** unter den Bedingungen der Heck-Reaktion liefern alkenylsubstituierte Verbindungen der Formel **9** (R¹¹ = -CR¹⁴=CR¹⁵-COOH, -CR¹⁴=CR¹⁵-COOAlkyl, -CR¹⁴=CR¹⁵-COOHaloalkyl, -CR¹⁴=CR¹⁵-CN) (S. Sengupta, S. Bhattacharya, J. Chem. Soc. Perkin Trans 1, 1993, 17, 1943). Reaktionsbedingungen für die Umsetzungen von Verbindungen der Formel **8** mit aromatischen Substraten unter Erhalt von Verbindungen der Formel **9** (R¹¹ = Aryl, Heteroaryl) werden in der Einleitung und in den folgenden Ausführungen vielfach beschrieben.

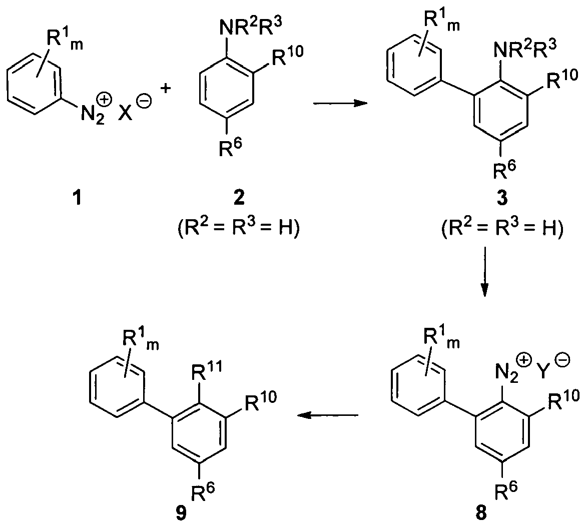

Dabei stehen
- R¹¹: für Wasserstoff, -OH, -SH, -SAlkyl, -SHaloalkyl, -SCycloalkyl, -S-(CH₂)_{q}-Aryl, -S-(CH₂)_{q}-Heteroaryl, -SAryl, -SHeteroaryl, Halogen, Cyano, -CR¹⁴=CR¹⁵-COOH -CR¹⁴=CR¹⁵-COOAlkyl, -CR¹⁴=CR¹⁵-COOHaloalkyl, -CR¹⁴=CR¹⁵-CN, -CR¹⁴=CR¹⁵-Aryl, -CR¹⁴=CR¹⁵-Heteroaryl, -SO₂R⁴, Aryl oder Heteroaryl;
- R¹⁴: für Wasserstoff, Alkyl oder Haloalkyl;
- R¹⁵: für Wasserstoff, Alkyl, Haloalkyl, -COOH, -COOAlykl, -COOHaloalkyl, Cyano, Aryl, Heteroaryl, -NHCOAlkyl oder -NHCOOAlkyl;
- Y⁻: für Halogenid, Hydrogensulfat, Sulfat, Tetrafluoroborat, Acetat, Trifluoracetat, Hexa-fluorophosphat, Hexafluoroantimonat, das Anion eines aromatischen 1,2-Dicarbonsäureimids oder das Anion eines aromatischen 1,2-Disulfonsäureimids;
und alle weiteren Reste sind wie oben definiert.

Im Rahmen der vorliegenden Erfindung haben die generisch verwendeten Begriffe die folgenden Bedeutungen:

Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder Iod, speziell Fluor, Chlor oder Brom, besonders bevorzugt Fluor oder Chlor.

Der Begriff "Alkyl" bezeichnet einen linearen oder verzweigten gesättigten Alkylrest, umfassend 1 bis 20 Kohlenstoffatome, wie Methyl, Ethyl, Propyl, 1-Methylethyl (Isopropyl), Butyl, 1-Methylpropyl (sec-Butyl), 2-Methylpropyl (Isobutyl), 1,1-Dimethylethyl (tert-Butyl), Pentyl, Hexyl, Heptyl, Octyl, 2-Ethylhexyl, Nonyl, Decyl oder 2-Propylheptyl und Stellungsisomere davon, bevorzugt Methyl, Ethyl oder Propyl.

Der Begriff "Haloalkyl", wie hierin und in den Haloalkyleinheiten von Haloalkoxy verwendet, beschreibt geradkettige oder verzweigte gesättigte Alkylgruppen mit 1 bis 10 Kohlenstoffatomen, wobei die Wasserstoffatome dieser Gruppen teilweise oder vollständig durch Halogenatome ersetzt sind. Beispiele hierfür sind Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl,1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2 Dichlor-2-fluorethyl, 2,2,2-Trichlorethyl, Pentafluorethyl, 3,3,3-Trifluorprop-1-yl, 1,1,1-Trifluorprop-2-yl, 3,3,3-Trichlorprop-1-yl, Heptafluorisopropyl, 1-Chlorbutyl, 2-Chlorbutyl, 3-Chlorbutyl, 4-Chlorbutyl, 1-Fluorbutyl, 2-Fluorbutyl, 3-Fluorbutyl, 4-Fluorbutyl und dergleichen, bevorzugt Fluormethyl, 2-Fluorethyl oder Trifluormethyl.

Der Begriff "Alkyliden" oder "Alkylen" bezeichnet über eine Doppelbindung gebundene Alkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind Methyliden (=CH₂), Ethyliden (=CHCH₃), 1-Propyliden (=CHCH₂CH₃) oder 2-Propyliden [=C(CH₃)₂].

Der Begriff "Cycloalkyliden" oder "Cycloalkylen" bezeichnet über eine Doppelbindung gebundene Cycloalkylreste, wobei der Cycloalkylrest gegebenenfalls 1, 2 oder 3

Substituenten trägt, die ausgewählt sind unter Halogen, Alkyl, Haloalkyl, Cycloalkyl, Alkoxy, Haloalkoxy. Beispiele sind Cyclopentyliden, Cyclohexyliden oder Cycloheptyliden.

Der Begriff "Haloalkyliden" oder "Haloalkylen" bezeichnet über eine Doppelbindung gebundene Haloalkylreste. Beispiele sind Fluormethylen (=CHF), 2-Chlorethyliden (=CH-CH₂C1), 3-Brom-1-propyliden (=CH₂-CH₂-CH₂Br).

Der Begriff "Arylalkyliden" oder "Arylalkylen" bezeichnet über eine Alkylideneinheit gebundene Arylreste, wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele sind Benzyliden (=CH-Phenyl), 1-Naphthyliden (=CH-Naphthyl) oder =CH-CH₂-Phenyl.

Der Begriff "Alkenyl" bezeichnet einen einfach ungesättigten, linearen oder verzweigten aliphatischen Rest mit 3 bis 8 Kohlenstoffatomen. Beispiele hierfür sind Propen-1-yl, Propen-2-yl (Allyl), But-1-en-1-yl, But-1-en-2-yl, But-1-en-3-yl, But-1-en-4-yl, But-2-en-1-yl, But-2-en-2-yl, But-2-en-4-yl, 2-Methylprop-1-en-1-yl, 2-Methylprop-2-en-1-yl und dergleichen, bevorzugt Propenyl oder But-1-en-4-yl.

Der Begriff "Cycloalkyl" bezeichnet einen gesättigten alicyclischen Rest mit 3 bis 10 Kohlenstoffatomen als Ringglieder. Beispiele sind Cyclopropyl, Cyclobutyl, Cyclopentyl, Cyclohexyl, Cycloheptyl, Cyclooctyl, Cyclononyl und Cyclodecyl. Die Cycloalkylreste können 1, 2 oder 3 Substituenten tragen, die ausgewählt sind unter Alkyl, Alkoxy oder Halogen, bevorzugt Cyclopropyl, Cyclobutyl, Cyclopentyl oder Cyclohexyl.

Der Begriff "Alkoxy" bezeichnet geradkettige oder verzweigte gesättigte Alkylgruppen, umfassend 1 bis 10 Kohlenstoffatome, die über ein Sauerstoffatom gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele für Alkoxy sind Methoxy, Ethoxy, n-Propoxy, 1-Methylethoxy (Isopropoxy), n-Butoxy, 1-Methylpropoxy (sec-Butoxy), 2-Methylpropoxy (Isobutoxy) und 1,1-Dimethylethoxy (tert-Butoxy), bevorzugt Methoxy, Ethoxy, n-Propoxy, oder -OCH₂-cyclo-Pentyl.

Der Begriff "Haloalkoxy" beschreibt geradkettige oder verzweigte gesättigte Haloalkylgruppen, umfassend 1 bis 10 Kohlenstoffatome, die über ein Sauerstoffatom gebunden sind. Beispiele hierfür sind Chlormethoxy, Brommethoxy, Dichlormethoxy, Trichlormethoxy, Fluormethoxy, Difluormethoxy, Trifluormethoxy, Chlorofluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, 1,1,2,2-Tetrafluorethoxy, 1-Chlor-1,2,2-trifluorethoxy, Pentafluorethoxy, 3,3,3-Trifluorprop-1-oxy, 1,1,1-Trifluorprop-2-oxy, 3,3,3-Trichlorprop-1-oxy, 1-Chlorbutoxy, 2-Chlorbutoxy, 3-Chlorbutoxy, 4-Chlorbutoxy, 1-Fluorbutoxy, 2-Fluorbutoxy, 3-Fluorbutoxy, 4-Fluorbutoxy und dergleichen, bevorzugt sind Fluormethoxy, Difluormethoxy oder Trifluormethoxy.

Der Begriff "Cycloalkoxy" bezeichnet einen Cycloalkylrest mit 3 bis 10 Kohlenstoffatomen als Ringglieder, die über ein Sauerstoffatom gebunden sind. Beispiele sind Cyclopropyloxy, Cyclobutyloxy, Cyclopentyloxy, Cyclohexyloxy, Cycloheptyloxy, Cyclooctyloxy, Cyclononyloxy und Cyclodecyloxy. Die Cycloalkylreste können 1, 2 oder 3 Substituenten tragen, die ausgewählt sind unter Alkyl und Halogen. Bevorzugte Cycloalkoxyreste sind Cyclopropyloxy, Cyclobutyloxy, Cyclopentyloxy oder Cyclohexyloxy.

Der Begriff "Alkylcarbonyl" bezeichnet über eine Carbonylgruppe gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylcarbonyl (Acetyl), Ethylcarbonyl, Propylcarbonyl, Isopropylcarbonyl, n-Butylcarbonyl, sec-Butylcarbonyl, Isobutylcarbonyl und tert-Butylcarbonyl, bevorzugt Methylcarbonyl oder Ethylcarbonyl.

Der Begriff "Haloalkylcarbonyl" bezeichnet über eine Carbonylgruppe gebundene Haloalkylreste mit 1 bis 10 Kohlenstoffatomen. Beispiele hierfür sind Fluormethylcarbonyl, Difluormethylcarbonyl, Trifluormethylcarbonyl, 1-Fluorethylcarbonyl, 2-Fluorethylcarbonyl, 1,1-Difluorethylcarbonyl, 2,2-Difluorethylcarbonyl, 2,2,2-Trifluorethylcarbonyl, Pentafluorethylcarbonyl und dergleichen, bevorzugt sind Fluormethylcarbonyl, Difluormethylcarbonyl oder Trifluormethylcarbonyl.

Der Begriff "Alkylcarbonyloxy" bezeichnet über eine Carbonyloxygruppe gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylcarbonyloxy (Acetoxy), Ethylcarbonyloxy, Propylcarbonyloxy und Isopropylcarbonyloxy, bevorzugt Methylcarbonyloxy oder Ethylcarbonyloxy.

Der Begriff "Haloalkylcarbonyloxy "bezeichnet über eine Carbonyloxygruppe gebundene Haloalkylreste mit 1 bis 10 Kohlenstoffatomen. Beispiele hierfür sind Fluormethylcarbonyloxy, Difluormethylcarbonyloxy, Trifluormethylcarbonyloxy, 1-Fluorethylcarbonyloxy, 2-Fluorethylcarbonyloxy, 1,1-Difluorethylcarbonyloxy, 2,2-Difluorethylcarbonyloxy, 2,2,2-Trifluorethylcarbonyloxy, Pentafluorethylcarbonyloxy und dergleichen, bevorzugt sind Fluormethylcarbonyloxy, Difluormethylcarbonyloxy oder Trifluormethylcarbonyloxy.

Der Begriff "Alkenylcarbonyl" bezeichnet über eine Carbonylgruppe gebundene Alkenylreste mit 3 bis 6 Kohlenstoffatomen, wobei der Alkenylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Propen-1-ylcarbonyl, Propen-2-ylcarbonyl (Allylcarbonyl), But-1-en-1-ylcarbonyl, But-1-en-2-ylcarbonyl, But-1-en-3-ylcarbonyl, But-1-en-4-ylcarbonyl, But-2-en-1-ylcarbonyl, But-2-en-2-ylcarbonyl, But-2-en-4-ylcarbonyl, 2-Methylprop-1-en-1-ylcarbonyl, 2-Methylprop-2-en-1-ylcarbonyl und dergleichen, bevorzugt sind Propen-1-ylcarbonyl, Propen-2-ylcarbonyl oder But-1-en-4-ylcarbonyl.

Der Begriff "Alkoxycarbonyl" bezeichnet über eine Carbonylgruppe gebundene Alkoxyreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methoxycarbonyl, Ethoxycarbonyl, Propoxycarbonyl, Isopropoxycarbonyl, n-Butoxycarbonyl, sec-Butoxycarbonyl, Isobutoxycarbonyl und tert-Butoxycarbonyl, bevorzugt sind Methoxycarbonyl, Ethoxycarbonyl, Propoxycarbonyl oder Isopropoxycarbonyl.

Der Begriff "Haloalkoxycarbonyl" bezeichnet über eine Carbonylgruppe gebundene Haloalkoxyreste mit 1 bis 10 Kohlenstoffatomen. Beispiele hierfür sind Fluormethoxycarbonyl, Difluormethoxycarbonyl, Trifluormethoxycarbonyl, 1-Fluorethoxycarbonyl, 2-Fluorethoxycarbonyl, 1,1-Difluorethoxycarbonyl, 2,2-Difluorethoxycarbonyl, 2,2,2-Trifluorethoxycarbonyl, Pentafluorethoxycarbonyl und dergleichen, bevorzugt sind Fluormethoxycarbonyl, Difluormethoxycarbonyl oder Trifluormethoxycarbonyl.

Der Begriff "Alkenyloxycarbonyl" bezeichnet Alkenyloxyreste mit 3 bis 8 Kohlenstoffatomen, die über eine Carbonylgruppe gebunden sind, wobei der Alkenylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Allyloxycarbonyl und Methallyloxycarbonyl, bevorzugt Allyloxycarbonyl.

Der Begriff "Alkylsulfonyl" bezeichnet über eine Sulfonylgruppe (SO₂) gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylsulfonyl, Ethylsulfonyl, Propylsulfonyl, Isopropylsulfonyl, n-Butylsulfonyl, sec-Butylsulfonyl, Isobutylsulfonyl und tert-Butylsulfonyl, bevorzugt Methylsulfonyl, Ethylsulfonyl, Propylsulfonyl oder Isopropylsulfonyl.

Der Begriff "Haloalkylsulfonyl" bezeichnet über eine Sulfonylgruppe (SO₂) gebundene Haloalkylreste mit 1 bis 10 Kohlenstoffatomen. Beispiele hierfür sind Fluormethylsulfonyl, Difluormethylsulfonyl, Trifluormethylsulfonyl, 1-Fluorethylsulfonyl, 2-Fluorethylsulfonyl, 1,1-Difluorethylsulfonyl, 2,2-Difluorethylsulfonyl, 2,2,2-Trifluorethylsulfonyl, Pentafluorethylsulfonyl und dergleichen, bevorzugt Fluormethylsulfonyl, Difluormethylsulfonyl oder Trifluormethylsulfonyl.

Der Begriff "Aryl", wie hierin und beispielsweise in den Arylalkyleinheiten von Arylalkyl verwendet, bezeichnet carbocyclische aromatische Reste mit 6 bis 14 Kohlenstoffatomen, wobei die Arylgruppe gegebenenfalls 1, 2, 3, 4 oder 5 Substituenten trägt, die ausgewählt sind unter Halogen, Cyano, Nitro, Alkyl, Haloalkyl, Alkoxy, Alkoxycarbonyl oder Haloalkoxy. Beispiele hierfür umfassen Phenyl, 4-Chlorphenyl, 4-Methoxyphenyl, Naphthyl, Fluorenyl, Azulenyl, Anthracenyl und Phenanthrenyl. Bevorzugt steht Aryl für Phenyl oder Naphthyl und insbesondere Phenyl.

Der Begriff "Heteroaryl", wie hierin und beispielsweise in den Heteroarylalkyleinheiten von Heteroarylalkyl verwendet, bezeichnet aromatische Reste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Nitro, Cyano, Alkyl, Haloalkyl, Alkoxy, Alkoxycarbonyl oder Haloalkoxy. Beispiele hierfür sind 5- und 6- gliedrige Heteroarylreste mit 1, 2, 3 oder 4 Heteroatomen ausgewählt unter O, N, S und SO₂ wie Pyrrolyl, 5-Methyl-2-pyrrolyl, Furanyl, 3-Methyl-2-furanyl, Thienyl, Pyrazolyl, Imidazolyl, Oxazolyl, Isoxazolyl, Thiazolyl, Isothiazolyl, Triazolyl, Tetrazolyl, Pyridyl, Pyrazinyl, Pyridazinyl, Pyrimidyl oder Triazinyl.

Der Begriff "Arylcarbonyl" bezeichnet Arylreste, die über eine Carbonylgruppe gebunden sind, wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind Phenylcarbonyl, 4-Nitrophenylcarbonyl, 2-Methoxyphenylcarbonyl, 4-Chlorphenylcarbonyl, 2,4-Dichlorphenylcarbonyl, 4-Nitrophenylcarbonyl oder Naphthylcarbonyl, bevorzugt Phenylcarbonyl.

Der Begriff "Arylalkyl" bezeichnet Arylreste, die über eine Alkylgruppe gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind 4-Methoxybenzyl, Benzyl, 2-Phenylethyl (Phenethyl) und dergleichen, bevorzugt Benzyl und Phenethyl.

Der Begriff "Alkylimino" bezeichnet einen Rest der Formel -N=R, der über den Stickstoff gebunden ist, worin R für Alkylen steht, wie =CH₂, =CHCH₃, =CHCH₂CH₃, =C(CH₃)₂, =CHCH₂CH₂CH₃, =C(CH₃)CH₂CH₃ oder =CHCH(CH₃)₂. Der Alkylenrest von "Alkylimino" trägt gegebenenfalls 1, 2 oder 3 Substituenten, die ausgewählt sind unter Halogen, Alkyl, Haloalkyl, Cycloalkyl, Alkoxy, Haloalkoxy.

Der Begriff "Arylalkylimino" bezeichnet einen Rest der Formel -N=R, der über den Stickstoff gebunden ist, worin R für Arylalkylen, wie Benzyliden (R = CH-Phenyl) steht. Die Arylgruppe in "Arylalkylimino" kann gegebenenfalls 1, 2, 3, 4 oder 5 Substituenten tragen, die ausgewählt sind unter Halogen, Alkyl, Haloalkyl, Alkoxy oder Haloalkoxy.

Der Begriff "Hydroxyalkyl" bezeichnet einen über eine Alkylgruppe gebundene Hydroxygruppe, wobei die Alkylgruppe gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind -CH₂OH, -(CH₂)₂OH oder -(CH₂)₃OH.

Der Begriff "Alkinyl" bezeichnet einen in Form einer Kohlenstoff-Kohlenstoff-Dreifachbindung zweifach ungesättigten, linearen oder verzweigten aliphatischen Rest mit 3 bis 8 Kohlenstoffatomen. Beispiele hierfür sind Propin-3-yl, But-1-in-1-yl, But-1-in-3-yl, But-1-in-4-yl, But-2-in-1-yl, But-2-in-4-yl und dergleichen, bevorzugt Propin-3-yl oder But-1-in-4-yl.

Der Begriff "Heteroarylalkyl" bezeichnet Heteroarylreste, die über eine Alkylgruppe gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 4-Pyridylmethyl, 1-(4-Pyridyl)ethyl, 2-(4-Pyridyl)ethyl, 2-Furanylmethyl, 1-(2-Furanyl)ethyl, 2-(2-Furanyl)ethyl und dergleichen, bevorzugt 4-Pyridyhmethyl.

Der Begriff "Alkoxyalkyl" bezeichnet über eine Alkylgruppe gebundene Alkoxyreste mit 1 bis 10 Kohlenstoffatomen, wobei die Alkylreste gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methoxymethyl, Ethoxymethyl, n-Propoxymethyl, Isopropoxymethyl, n-Butoxymethyl, sec-Butoxymethyl, Isobutoxymethyl, tert-Butoxymethyl, Methoxyethyl, Ethoxyethyl, Propoxyethyl, Isopropoxyethyl, n-Butoxyethyl, sec-Butoxyethyl, Isobutoxyethyl, tert-Butoxyethyl und dergleichen, bevorzugt sind Methoxymethyl, Ethoxymethyl, n-Propoxymethyl, Isopropoxymethyl, Methoxyethyl, Ethoxyethyl, Propoxyethyl oder Isopropoxyethyl.

Der Begriff "Aryloxyalkyl" bezeichnet über eine Alkylgruppe gebundene Aryloxyreste mit 6 bis 14 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Die Arylgruppe in "Aryloxyalkyl" trägt gegebenenfalls 1, 2, 3, 4 oder 5 Substituenten, die ausgewählt sind unter Halogen, Alkyl, Haloalkyl, Alkoxy oder Haloalkoxy. Beispiele hierfür sind Phenoxymethyl, Phenoxyethyl, Phenoxypropyl, Phenoxybutyl, 1-Naphtyloxymethyl, 1-(1-Naphtyloxy)ethyl, 2-(1-Naphtyloxy)ethyl, 1-(1-Naphtyloxy)propyl, 2-(1-Naphtyloxy)propyl, 3-(1-Naphtyloxy)propyl und dergleichen, bevorzugt sind Phenoxymethyl und Phenoxyethyl.

Der Begriff "Heteroaryloxyalkyl" bezeichnet über eine Alkylgruppe gebundene Heteroaryloxyreste mit 1 bis 4 Heteroatomen, die ausgewählt sind unter O, N, S und SO₂, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Die Heteroarylgruppe in "Heteroaryloxyalkyl" trägt gegebenenfalls 1, 2, 3 oder 4 Substituenten, die ausgewählt sind unter Halogen, Alkyl, Haloalkyl, Alkoxy oder Haloalkoxy. Beispiele hierfür sind 4-Pyridyloxymethyl, 1-(4-Pyridyloxy)ethyl, 2-(4-Pyridyloxy)ethyl, 1-(4-Pyridyloxy)propyl, 2-(4-Pyridyloxy)propyl, 3-(4-Pyridyloxy)propyl, 2-Furanyloxymethyl, 1-(2-Furanyloxy)ethyl, 2-(2-Furanyloxy)ethyl, 1-(2-Furanyloxy)propyl, 2-(2-Furanyloxy)propyl, 3-(2-Furanyloxy)propyl und dergleichen, bevorzugt sind 4-Pyridyloxymethyl oder 1-(4-Pyridyloxy)ethyl.

Der Begriff "Aminoalkyl" bezeichnet einen über eine Alkylgruppe gebundenen -NH₂-Rest, wobei die Alkylgruppe gegebenenfalls 1, 2 oder 3 Substituenten trägt, die ausgewählt sind unter Halogen, Alkyl, Haloalkyl, Cycloalkyl, Alkoxy, Haloalkoxy. Beispiele hiefür sind Aminomethyl [-CH₂NH₂], Aminoethyl [-(CH₂)₂NH₂] und dergleichen, bevorzugt sind -CH₂NH₂, -(CH₂)₂NH₂ oder -(CH₂)₃NH₂.

Der Begriff "Alkylaminoalkyl" bezeichnet einen über eine Alkylgruppe gebundenen -NHR⁴ oder -NR⁴R⁵ -Rest, wobei R⁴ und R⁵ wie oben definiert sind und die Alkylgruppe gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind Methylaminomethyl [-CH₂-NH-CH₃], N,N-Dimethylaminomethyl [-CH₂-N(CH₃)₂], N,N-Dimethylaminoethyl [-(CH₂)₂-N(CH₃)₂] und dergleichen, bevorzugt -CH₂-N(CH₃)₂ oder -(CH₂)₂-N(CH₃)₂.

Der Begriff "Cycloalkylcarbonyl" bezeichnet über eine Carbonylgruppe gebundene Cycloalkylreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropylcarbonyl, Cyclobutylcarbonyl, Cyclopentylcarbonyl, Cyclohexylcarbonyl und dergleichen, bevorzugt Cyclopropylcarbonyl, Cyclopentylcarbonyl oder Cylcohexylcarbonyl.

Der Begriff "Arylalkylcarbonyl" bezeichnet über eine Carbonylgruppe gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind Benzylcarbonyl, 2-Phenylethylcarbonyl und dergleichen, bevorzugt Benzylcarbonyl oder 2-Phenylethylcarbonyl.

Der Begriff "Heteroarylalkylcarbonyl" bezeichnet über eine Carbonylgruppe gebundene Heteroarylalkylreste mit 1 bis 4 Heteroatomen, die ausgewählt sind unter O, N, S und SO₂, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 4-Pyridylmethylcarbonyl, 1-(4-Pyridyl)ethylcarbonyl, 2-Furanylmethylcarbonyl, 1-(2-Furanyl)ethylcarbonyl und dergleichen, bevorzugt 4-Pyridylmethylcarbonyl.

Der Begriff "Cycloalkoxycarbonyl" bezeichnet über eine Carbonylgruppe gebundene Cycloalkoxyreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropyloxycarbonyl, Cyclobutyloxycarbonyl, Cyclopentyloxycarbonyl, Cyclohexyloxycarbonyl, Cycloheptyloxycarbonyl, Cyclooctyloxycarbonyl, Cyclononyloxycarbonyl und dergleichen, bevorzugt sind Cyclopropyloxycarbonyl, Cyclopentyloxycarbonyl oder Cyclohexyloxycarbonyl.

Der Begriff "Arylalkoxycarbonyl" bezeichnet über eine Carbonylgruppe gebundene Arylalkoxyreste mit 6 bis 14 Kohlenstoffatomen, wobei der Alkoxyrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzyloxycarbonyl, 2-Phenylethyloxycarbonyl und der gleichen, bevorzugt ist Benzyloxycarbonyl.

Der Begriff "Aryloxy" bezeichnet einen Arylrest, der über ein Sauerstoffatom gebunden ist, wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind Phenyloxy (Phenoxy), Naphtyloxy, Fluorenyloxy und der gleichen, bevorzugt ist Phenoxy.

Der Begriff "Aryloxycarbonyl" bezeichnet über eine Carbonylgruppe gebundene Aryloxyreste mit 6 bis 14 Kohlenstoffatomen, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenyloxycarbonyl (Phenoxycarbonyl), Naphtyloxycarbonyl, Fluorenyloxycarbonyl und dergleichen, bevorzugt ist Phenoxycarbonyl.

Der Begriff "Heteroaryloxy" bezeichnet über ein Sauerstoffatom gebundene Heteroarylreste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Pyrrolyloxy, Furanyloxy, Thienyloxy, Pyrazolyloxy, Imidazolyloxy, Oxazolyloxy, Thiazolyloxy, Pyridyloxy, Pyrazinyloxy, Pyridazinyloxy, Pyrimidyloxy und dergleichen, bevorzugt Pyrazolyloxy oder Pyridyloxy.

Der Begriff "Heteroaryloxycarbonyl" bezeichnet über eine Carbonylgruppe gebundene Heteroaryloxyreste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Pyrrolyloxycarbonyl, Furanyloxycarbonyl, Thienyloxycarbonyl, Pyrazolyloxycarbonyl, Imidazolyloxycarbonyl, Oxazolyloxycarbonyl, Thiazolyloxycarbonyl, Pyridyloxycarbonyl, Pyrazinyloxycarbonyl, Pyridazinyloxycarbonyl, Pyrimidyloxycarbonyl und der gleichen, bevorzugt Imidazolyloxycarbonyl oder Oxazolyloxycarbonyl.

Der Begriff "Arylalkyloxy" bezeichnet über ein Sauerstoffatom gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzyloxy, 2-Phenylethyloxy (Phenethyloxy) und dergleichen, bevorzugt ist Benzyloxy.

Der Begriff "Cycloalkylimino" bezeichnet einen Rest der Formel -N=R, der über den Stickstoff gebunden ist, worin R für Cycloalkylidenreste steht, die gegebenenfalls 1, 2 oder 3 Substituenten tragen, die ausgewählt sind unter Halogen, Alkyl, Haloalkyl, Cycloalkyl, Alkoxy, Haloalkoxy. Beispiele für R sind Cyclopentyliden, Cyclohexyliden, Cycloheptyliden und dergleichen.

Der Begriff "Haloalkylimino" bezeichnet einen Rest der Formel -N=R, der über den Stickstoff gebunden ist, worin R für Haloalkylidenreste steht, wobei die Wasserstoffatome dieser geradkettige oder verzweigte Alkylengruppen teilweise oder vollständig durch Halogenatome ersetzt sind. Beispiele für R sind Chlormethylen, Brommethylen, Dichlormethylen, Fluormethylen, Difluormethylen, Chlorofluormethylen, 1-Chlorethylen, 1-Bromethylen, 1-Fluorethylen, 2-Fluorethylen, 2,2-Difluorethylen, 2,2,2-Trifluorethylen, 2-Chlor-2-fluorethylen, 2-Chlor-2,2-difluorethylen, 2,2-Dichlor-2-fluorethylen, 2,2,2-Trichlorethylen und dergleichen.

Der Begriff "Cycloalkylcarbonyloxy" bezeichnet über eine Carbonyloxygruppe gebundene Cycloalkylreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropylcarbonyloxy, Cyclobutylcarbonyloxy, Cyclopentylcarbonyloxy, Cyclohexylcarbonyloxy, Cycloheptylcarbonyloxy, Cyclooctylcarbonyloxy, Cyclononylcarbonyloxy und dergleichen, bevorzugt Cyclopentylcarbonyloxy oder Cyclohexylcarbonyloxy.

Der Begriff "Arylalkylcarbonyloxy" bezeichnet über eine Carbonyloxygruppe gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzylcarbonyloxy, 2-Phenylethylcarbonyloxy (Phenethylcarbonyloxy) und dergleichen, bevorzugt Benzylcarbonyloxy.

Der Begriff "Arylcarbonyloxy" bezeichnet über eine Carbonyloxygruppe gebundene Arylreste, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenylcarbonyloxy, Naphthylcarbonyloxy, Fluorenylcarbonyloxy, Anthracenylcarbonyloxy und dergleichen, bevorzugt Phenylcarbonyloxy.

Der Begriff "Heteroarylcarbonyloxy" bezeichnet über eine Carbonyloxygruppe gebundene Heteroarylreste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylcarbonyloxy, 2-Furanylcarbonyloxy, 2-Thienylcarbonyloxy, 3-Pyrazolylcarbonyloxy, 2-Imidazolylcarbonyloxy, 2-Oxazolylcarbonyloxy, 2-Thiazolylcarbonyloxy, 4-Triazolylcarbonyloxy, 4-Pyridylcarbonyloxy und dergleichen.

Der Begriff "Heteroarylcarbonyl" bezeichnet über eine Carbonylgruppe gebundene Heteroarylreste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylcarbonyl, 2-Furanylcarbonyl, 2-Thienylcarbonyl, 3-Pyrazolylcarbonyl, 2-Imidazolylcarbonyl, 2-Oxazolylcarbonyl, 2-Thiazolylcarbonyl, 4-Triazolylcarbonyl, 4-Pyridylcarbonyl und dergleichen.

Der Begriff "Alkylthio" bezeichnet Alkylreste, die über ein Schwefelatom gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylthio, Ethylthio, n-Propylthio, 1-Methylethylthio (Isopropylthio), n-Butylthio, 1-Methylpropylthio (sec-Butylthio), 2-Methylpropylthio (Isobutylthio) und 1,1-Dimethylethylthio (tert-Butylthio) und der gleichen, bevorzugt Methylthio, Ethylthio oder n-Propylthio.

Der Begriff "Haloalkylthio" beschreibt Haloalkylgruppen, die über ein Schwefelatom gebunden sind. Beispiele hierfür sind Chlormethylthio, Brommethylthio, Dichlormethylthio, Trichlormethylthio, Fluormethylthio, Difluormethylthio, Trifluormethylthio, Chlorofluormethylthio, Dichlorfluormethylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, 1,1,2,2-Tetrafluorethylthio, 1-Chlor-1,2,2-trifluorethylthio, Pentafluorethylthio, 3,3,3-Trifluorprop-1-ylthio, 1,1,1-Trifluorprop-2-ylthio, 3,3,3-Trichlorprop-1-ylthio, 1-Chlorbutylthio, 2-Chlorbutylthio, 3-Chlorbutylthio, 4-Chlorbutylthio, 1-Fluorbutylthio, 2-Fluorbutylthio, 3-Fluorbutylthio, 4-Fluorbutylthio und dergleichen, bevorzugt sind Fluormethylthio, 2-Fluorethylthio oder Trifluormethylthio.

Der Begriff "Cycloalkylthio" bezeichnet Cycloalkylreste, die über ein Schwefelatom gebunden sind. Beispiele sind Cyclopropylthio, Cyclobutylthio, Cyclopentylthio, Cyclohexylthio, Cycloheptylthio, Cyclooctylthio, Cyclononylthio und Cyclodecylthio. Die Cycloalkylreste können 1, 2 oder 3 Substituenten tragen, die ausgewählt sind unter Alkyl und Halogen. Bevorzugte Cycloalkylthioreste sind Cyclopentylthio oder Cyclohexylthio.

Der Begriff "Arylthio" bezeichnet Arylreste, die über ein Schwefelatom gebunden sind, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenylthio, Naphtylthio, Fluorenylthio und der gleichen, bevorzugt Phenylthio.

Der Begriff "Heteroarylthio" bezeichnet über ein Schwefelatom gebundene Heteroarylreste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylthio, 3-Furanylthio, 3-Thienylthio, 2-Pyridylthio und dergleichen, bevorzugt 2-Pyridylthio oder 4-Pyridylthio.

Der Begriff "Arylalkylthio" bezeichnet über ein Schwefelatom gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzylthio, 2-Phenylethylthio und dergleichen, bevorzugt Benzylthio.

Der Begriff "Cycloalkylsulfonyl" bezeichnet über eine Sulfonylgruppe (SO₂) gebundene Cycloalkylreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropylsulfonyl, Cyclobutylsulfonyl, Cyclopentylsulfonyl, Cyclohexylsulfonyl und dergleichen, bevorzugt Cyclopropylsulfonyl, Cyclopentylsulfonyl oder Cyclohexylsulfonyl.

Der Begriff "Arylsulfonyl" bezeichnet über eine Sulfonylgruppe (SO₂) gebundene Arylreste, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenylsulfonyl, Naphtylsulfonyl, Fluorenylsulfonyl und dergleichen, bevorzugt Phenylsulfonyl.

Der Begriff "Heteroarylsulfonyl" bezeichnet über eine Sulfonylgruppe (SO₂) gebundene Heteroarylreste mit 1 bis 4 Heteroatomen, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylsulfonyl, 2-Furanylsulfonyl, 2-Thienylsulfonyl, 3-Pyrazolylsulfonyl, 2-Imidazolylsulfonyl, 2-Oxazolylsulfonyl, 4-Pyridylsulfonyl und dergleichen, 2-Pyrrolylsulfonyl, 2-Furanylsulfonyl oder 4-Pyridylsulfonyl.

Der Begriff "Arylalkylsulfonyl" bezeichnet über eine Sulfonylgruppe (SO₂) gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzylsulfonyl, 2-Phenylethylsulfonyl und dergleichen, bevorzugt Benzylsulfonyl.

Die nachfolgend gemachten Ausführungen zu bevorzugten Ausgestaltungen der erfindungsgemäßen Verfahren, insbesondere zu bevorzugten Ausgestaltungen der Reste der verschiedenen Edukte und Produkte und der Reaktionsbedingungen der erfindungsgemäßen Verfahren, gelten sowohl allein für sich genommen als auch insbesondere in jeder denkbaren Kombination miteinander.

Die hierin beschriebenen Umsetzungen werden in für derartige Reaktionen üblichen Reaktionsgefäßen durchgeführt, wobei die Reaktionsführung sowohl kontinuierlich, semikontinuierlich als auch diskontinuierlich ausgestaltet werden kann. In der Regel wird man die jeweiligen Reaktionen unter Atmosphärendruck durchführen. Die Reaktionen können jedoch auch unter vermindertem (z.B. 0.1 bis 1.0 bar) oder erhöhtem Druck (z. B. 1.0 bis 100 bar) durchgeführt werden.

Insbesondere ist es bevorzugt die Ausführungsformen in jeder beliebigen Kombination miteinander zu kombinieren.

Im Rahmen der vorliegenden Erfindung steht m bevorzugt für 0, 1, 2, 3 oder 4, insbesondere für 0, 1, 2 oder 3, besonders bevorzugt für 1, 2 oder 3.

Im Rahmen der vorliegenden Erfindung steht n bevorzugt für 1, 2, 3, 4 oder 5 insbesondere für 1, 2 oder 3.

Im Rahmen der vorliegenden Erfindung steht q bevorzugt für 1, 2, 3 oder 4, insbesondere für 1, 2 oder 3.

Im Rahmen der vorliegenden Erfindung steht R¹ bevorzugt für Halogen, Alkyl, Hydroxyalkyl, Haloalkyl, Alkoxy, Haloalkoxy, Nitro, Cyano, Aryl, Aryloxy oder Heteroaryl. Besonders bevorzugt steht R¹ für Halogen, Alkoxy, Haloalkoxy oder gegebenenfalls mit Halogen, Alkyl oder Alkoxy substituiertes Aryloxy und stärker bevorzugt für Chlor, Brom, Fluor, Alkoxy oder Phenoxy (besonders bevorzugt für Chlor, Brom, Fluor oder Methoxy). Insbesondere steht R¹ für 2-Me, 3-Me, 4-Me, 2-F, 3-F, 4-F, 2-Cl, 3-Cl, 4-Cl, 2-Br, 3-Br, 4-Br, 2-Methoxy, 3-Methoxy, 4-Methoxy, 2-CF₃, 3-CF₃, 4-CF₄, 2-OCF₃, 3-OCF₃, oder 4-OCF₃. Die Positionsangaben beziehen sich dabei auf die 1-Position, über die der sich von der Verbindung der Formel 1 ableitende Arylrest an den Anilinring der Verbindung der Formel 3 gebunden ist bzw. auf die 1-Position des Diazoniumrestes in der Verbindung der Formel 1.

Im Rahmen der vorliegenden Erfindung steht X⁻ bevorzugt für ein Halogenid, wie Fluorid, Chlorid, Bromid, Iodid, BF₄⁻, PF₆⁻, Hydrogensulfat, Sulfat (½ SO₄²⁻), Acetat, das Anion eines aromatischen 1,2-Dicarbonsäureimids oder das Anion eines aromatischen 1,2-Disulfonimids. Das Anion entsteht in den beiden zuletzt genannten Fällen durch Abstraktion des Protons am Imid-Stickstoffatom. Besonders bevorzugt steht X⁻ für ein Halogenid, wie Chlorid, Bromid, BF⁻₄ oder Sulfat (½SO₄²⁻).

Im Rahmen der vorliegenden Erfindung steht Y⁻ bevorzugt für ein Halogenid, wie Fluorid, Chlorid, Bromid, Iodid, BF₄⁻, PF₆⁻, Hydrogensulfat, Sulfat (½ SO₄²⁻), Acetat, das Anion eines aromatischen 1,2-Dicarbonsäureimids oder das Anion eines aromatischen 1,2-Disulfonimids. Das Anion entsteht in den beiden zuletzt genannten Fällen durch Abstraktion des Protons am Imid-Stickstoffatom. Besonders bevorzugt steht Y⁻ für ein Halogenid, wie Chlorid, Bromid, BF₄⁻ oder Sulfat (½ SO₄²⁻).

Im Rahmen der vorliegenden Erfindung steht R² bevorzugt für Wasserstoff, Alkyl, Haloalkyl, Hydroxyalkyl, Cycloalkyl, Aryl, Heteroaryl, Arylalkyl oder Heteroarylalkyl. Besonders bevorzugt steht R² für Wasserstoff oder C₁-C₆ Alkyl; insbesondere für Wasserstoff.

Im Rahmen der vorliegenden Erfindung steht R³ bevorzugt für Wasserstoff, Alkyl, Haloalkyl, Hydroxyalkyl, Cycloalkyl, Aryl, Heteroaryl, Arylalkyl oder Heteroarylalkyl. Besonders bevorzugt steht R³ für Wasserstoff oder C₁-C₆ Alkyl; insbesondere für Wasserstoff.

Weiter bevorzugt bilden R² und R³ zusammen mit dem Stickstoffatom, an das sie gebunden sind, einen 5 oder 6 gliedrigen Ring mit 5 oder 6 Ringatomen, ausgewählt aus O, S, N und C.

Weiter bevorzugt bilden R² und R³ zusammen einen Alkylidenrest.

Am stärksten bevorzugt sind R² und R³ Wasserstoffatome.

Im Rahmen der vorliegenden Erfindung steht R⁴ bevorzugt für Wasserstoff, Alkyl, Haloalkyl, Cycloalkyl, Arylalkyl, Aryl oder Heteroaryl.

Im Rahmen der vorliegenden Erfindung steht R⁵ bevorzugt für Wasserstoff, Alkyl, Haloalkyl, Cycloalkyl, Arylalkyl, Aryl oder Heteroaryl.

Im Rahmen der vorliegenden Erfindung steht R¹⁰ bevorzugt für Wasserstoff, Halogen, Alkyl, Haloalkyl, Alkoxy, Haloalkoxy, Cycloalkyl, Aryl oder Heteroaryl. Besonders bevorzugt steht R¹⁰ für Wasserstoff, Halogen, C₁-C₆ Alkyl oder C₁-C₆ Haloalkyl. Insbesondere bevorzugt steht R¹⁰ für Wasserstoff.

Im Rahmen der vorliegenden Erfindung steht R¹¹ bevorzugt für Wasserstoff, Halogen, Hydroxy, Cyano, Aryl oder Heteroaryl.

Im Rahmen der vorliegenden Erfindung steht R¹⁴ bevorzugt für Wasserstoff, Alkyl oder Haloalkyl.

Im Rahmen der vorliegenden Erfindung steht R¹⁵ bevorzugt für Wasserstoff, Alkyl, Haloalkyl, Cyano, Aryl oder Heteroaryl.

Im Rahmen der vorliegenden Erfindung steht R⁶ bevorzugt für Wasserstoff, Halogen, Alkyl, Haloalkyl, Cycloalkyl, Alkoxy, Cyano, Haloalkoxy, Cycloalkoxy, Alkylcarbonyloxy, Haloalkylcarbonyloxy, Aryloxy, Aryl oder Heteroaryl. Für R⁶ besonders bevorzugt sind: Wasserstoff, Fluor, Chlor, Brom, Cyano, Methyl, Ethyl, -OMe, -OCH₂F, -OCHF₂, -OCF₃, -OCF₂Cl, -OEt, -OCF₂CHClF, -OCF₂CHF₂, -O(CH₂)₂Cl, -OCH₂CF₃, -OCF₂CF₃, -O(CH₂)₂CH₃, -OCH(CF₃)₂, -O(CH₂)₂OMe, -OCHMe₂, -O(CH₂)₂CN, -O(CH₂)₃CH₃, -OCMe₃, -OCH(Me)Et, -OCH₂CHMe₂, -O(CH₂)₂OEt, -OCH(Me)CH₂OMe, -O(CH₂)₄CH₃, -O(CH₂)₂CHMe₂, -OCHEt₂, -OCH₂CMe₃, -O(CH₂)₂O(CH₂)₂Me, -O(CH₂)₂O(CH₂)₂OMe, -O(CH₂)₅Me, -O(CH₂)₆Me, -OCH(Me)(CH₂)₄Me, -O(CH₂)₇Me, -O(CH₂)₈Me, -O(CH₂)₉Me, -O(CH₂)₁₁Me, -O(CH₂)₁₃Me, -O(CH₂)₁₅ Me, -O(CH₂)₁₇Me. Insbesondere bevorzugt ist R⁶ Wasserstoff, Fluor, Chlor, Brom, Methoxy, CN oder Ethoxy.

Vorzugsweise sind solche Verfahrensmaßnahmen ausgewählt unter folgenden:
- Durchführung in wenigstens einem Lösungsmittel bzw. Lösungsmittelsystem;
- Durchführung in wenigstens einem Lösungsmittel bzw. Lösungsmittelsystem, das die radikalische Zersetzung der Verbindung der Formel 1 in Stickstoff und ein Arylradikal bewirkt und/oder die Umsetzung in einer anderen Art und Weise begünstigt;
- Durchführung in wenigstens einem wässrigen Lösungsmittel bzw. Lösungsmittelsystem, wobei das Reaktionsgemisch einen pH-Wert von größer als 9 aufweist;
- Durchführung in wenigstens einem Lösungsmittel bzw. Lösungsmittelsystem im basischen Bereich;
- Durchführung in wenigstens einem Lösungsmittel bzw. Lösungsmittelsystem und in Gegenwart wenigstens einer Base;
- Durchführung in Gegenwart wenigstens eines Reduktionsmittels;
- Durchführung unter Bestrahlung mit elektromagnetischer Strahlung im sichtbaren und/oder ultravioletten Bereich;
- Durchführung unter Anwendung von Ultraschall;
- Durchführung unter den Bedingungen einer elektrochemischen Reduktion;
- Durchführung unter Radiolysebedingungen;
- und einer Kombination von wenigstens zwei dieser Maßnahmen.

Der Begriff "Reduktionsmittel" bezeichnet diejenigen Elemente und Verbindungen, die als Elektronendonatoren [auch Elektronen-Donator-Komplexe] bestrebt sind, durch die Abgabe von Elektronen in einen energieärmeren Zustand überzugehen, v. a. unter Bildung stabiler Elektronenschalen. Ein Maß für die Stärke eines Reduktionsmittels ist das Redoxpotential. Beispiele für Reduktionsmittel sind anorganische Salze, Metalle, Metallsalze oder reduzierende organische Verbindungen.

Wenn die Reaktion in Gegenwart eines Reduktionsmittels durchgeführt wird, so erfolgt die Durchführung vorzugsweise so, dass die Verbindung der Formel 2 und das Reduktionsmittel, vorzugsweise in einem Lösungsmittel oder Lösungsmittelsystem gelöst oder dispergiert, vorgelegt und mit der Verbindung der Formel 1, sukzessive versetzt werden. Bezüglich Zugabegeschwindigkeit, Reaktionstemperatur und Lösungsmittel oder Lösungsmittelsystem wird auf die folgenden Ausführungen verwiesen.

Das wenigstens eine Reduktionsmittel ist vorzugsweise ausgewählt unter reduzierenden Metallsalzen, Metallen und/oder reduzierenden Anionen; geeignet sind jedoch auch andere Reduktionsmittel, deren Reduktionspotential ausreichend groß ist, um auf die jeweils eingesetzte Verbindung der Formel **1** ein Elektron zu übertragen. Dazu gehören so unterschiedliche Verbindungen, wie Pyren, Ascorbinsäure und Hämoglobin. Bevorzugt ist jedoch die Verwendung von reduzierenden Metallen, Metallsalzen und/oder reduzierenden

### Anionen.

Im Rahmen der vorliegenden Erfindung können beliebige reduzierende Metallsalze verwendet werden, so lange ihr Reduktionspotential ausreichend groß ist, um auf die jeweils eingesetzte Verbindung der Formel **1** ein Elektron zu übertragen.

Unter reduzierenden Metallsalzen versteht man im Rahmen der vorliegenden Erfindung solche, in denen unter den Reaktionsbedingungen die stabilste Oxidationszahl des Metalls höher ist als in der eingesetzten Form, so dass das Metallsalz als Reduktionsmittel wirkt.

Bevorzugte Metallsalze sind im Reaktionsmedium wenigstens teilweise löslich. Da das Reaktionsmedium vorzugsweise wässrig ist, sind bevorzugte reduzierende Metallsalze dementsprechend wasserlöslich. Bevorzugte Gegenanionen der Metallsalze sind übliche wasserlösliche Anionen, wie die Halogenide, insbesondere Chlorid, Sulfat, Nitrat, Acetat und dergleichen.

Geeignet sind aber auch Metallkomplexe, wie Hexacyanoferrat(II) oder Ferrocen.

Reduzierende Metallsalze sind ausgewählt unter Cu(I)-Salzen, Fe(II)-Salzen, Zinn(II)-Salzen und Vanadium(II)-Salzen und insbesondere unter Cu(I)-Salzen und Fe(II)-Salzen. Hierunter bevorzugt sind deren wasserlösliche Salze, wie die Chloride, Sulfate, Nitrate, Acetate und dergleichen.

Bevorzugte reduzierende Metalle sind ausgewählt unter Eisen, Kupfer, Cobalt, Nickel, Zink, Magnesium, Titan und Chrom, besonders bevorzugt Eisen und Kupfer.

Vorzugsweise setzt man das/die reduzierende(n) Metall(e) oder Metallsalz(e) in einer Gesamtmenge von 0,005 bis 8 Mol, besonders bevorzugt von 0,01 bis 3 Mol, stärker bevorzugt von 0,1 bis 1 Mol bezogen auf 1 Mol der Verbindung der Formel **1** ein.

Wird die Reaktion in entgasten (d.h. von Sauerstoff befreiten) Lösungsmitteln oder Lösungsmittelsystemen und unter einer Inertgasatmosphäre, wie Stickstoff oder Argon, durchgeführt, so kann das reduzierende Metallsalz in geringeren Mengen eingesetzt werden, beispielsweise in einer Menge von 0,005 bis 4 Mol bezogen auf 1 Mol der Verbindung der Formel **1.**

Geeignete reduzierende Anionen sind beispielsweise Bromid, Iodid, Sulfit, Hydrogensulfit, Pyrosulfit, Dithionit, Thiosulfat, Nitrit, Phosphit, Hypophosphit, ArS⁻, Xanthogenate (R'OCS₂⁻; R' = Alkyl, Aryl), Alkoxide, wie Methanolat, Ethanolat, Propanolat, Isopropanolat, Butanolat, Isobutanolat und tert-Butanolat, und Phenoxid. Die reduzierenden Anionen selbstverständlich vorzugsweise unter solchen ausgewählt, deren Reduktionspotential auch im gewählten pH-Bereich noch ausreicht, um die Zersetzung der Verbindung der Formel **1** in ein Arylradikal und Stickstoff zu bewirken.

Die reduzierenden Anionen werden in einer Menge von vorzugsweise 0,005 bis 8 Mol besonders bevorzugt von 0,01 bis 6 Mol und insbesondere von 1 bis 6 Mol, bezogen auf 1 Mol der Verbindung der Formel **1** eingesetzt.

Alternativ oder zusätzlich erfolgt in einer bevorzugten Ausführungsform des erfindungsgemäßen Verfahrens die Durchführung unter den Bedingungen einer elektrochemischen Reduktion. Bei dieser Vorgehensweise werden aus der Verbindung der Formel **1** durch kathodische Reduktion Aryldiazenylradikale generiert, was den Zerfall der oben genannten Verbindungen initiiert.

Die Durchführung erfolgt beispielsweise so, dass in das Reaktionsgefäß, welches die in einem geeigneten Lösungsmittel oder Lösungsmittelsystem vorgelegte Verbindung der Formel **2** enthält, Kathode und Anode angeordnet werden und während der sukzessiven Zugabe der Verbindung der Formel **1** Spannung angelegt wird. Die zu wählende Spannung und Stromdichte hängt von diversen Faktoren, wie Zugabegeschwindigkeit und Lösungsmittel bzw. Lösungsmittelsystem ab und muss im Einzelfall bestimmt werden, was beispielsweise mit Hilfe von Vorversuchen gelingt. Die Lösungsmittel oder Lösungsmittelsysteme werden geeigneterweise so gewählt, dass sie unter den gegebenen Reaktionsbedingungen möglichst keine Konkurrenzreaktion an den Elektroden eingehen. Da die kathodische Reduktion von Protonen auch bei sehr geringen Stromdichten und Spannung nur schwer zu vermeiden ist, werden bevorzugt nichtprotische, polare Lösungsmittel, wie Acetonitril, Dimethylformamid oder Aceton, eingesetzt.

Von den genannten Maßnahmen ist die Durchführung in Gegenwart wenigstens eines Reduktionsmittels und insbesondere wenigstens eines reduzierenden Anions abhängig von den Reaktionsbedingungen bevorzugt.

Alternativ oder zusätzlich erfolgt das Verfahren zur Herstellung von Verbindungen der Struktur **3** dadurch, dass die Reaktion unter Bestrahlung mit elektromagnetischer Strahlung im sichtbaren und/oder ultravioletten Bereich erfolgt. Bevorzugt wird elektromagnetische Strahlung mit einer Wellenlänge im Bereich von 100 bis 400 nm, besonders bevorzugt im Bereich von 200 bis 380 nm und insbesondere im Bereich von 250 bis 360 nm eingesetzt.

Die Durchführung unter Bestrahlung erfolgt bevorzugt in der Weise, dass die Verbindung der Formel 2 in einem geeigneten Lösungsmittel oder Lösungsmittelsystem vorgelegt wird und während der sukzessiven Zugabe der Verbindung der Formel 1 unter Kühlen bestrahlt wird. Insbesondere wenn UV-Strahlung verwendet wird, werden die Lösungsmittel oder Lösungsmittelsysteme vorzugsweise in entgaster Form eingesetzt, da ansonsten Sauerstoffradikale entstehen können, die zu unerwünschten Produkten führen können. Da sich Wasser oder wässrige Lösungen nicht trivial entgasen lassen, bieten sich in diesem Fall die unten genannten organischen Lösungsmittel an.

Alternativ oder zusätzlich erfolgt das Verfahren zur Herstellung von Verbindungen der Formel **3** dadurch, dass die Reaktion unter Anwendung von Ultraschall durchgeführt wird. Wie alle Schallwellen erzeugt auch Ultraschall eine periodische Kompression und Dehnung des Mediums; die Moleküle werden zusammengedrückt und gedehnt. Es bilden sich kleine Bläschen, die anwachsen und sofort wieder implodieren. Dieses Phänomen wird Kavitation genannt. Jedes implodierende Bläschen sendet Schockwellen und winzige Flüssigkeitsstrahlen mit einer Geschwindigkeit von etwa 400 km/h aus, die auf die nähere Umgebung einwirken. Kavitation kann beispielsweise ausgenutzt werden, um chemische Reaktionen zu beschleunigen und die Löslichkeit von Produkten in einem bestimmten Medium zu erhöhen.

Die Durchführung unter Anwendung von Ultraschall kann beispielsweise so erfolgen, dass sich das Reaktionsgefäß, in welchem die Verbindung der Formel **2** in einem geeigneten Lösungsmittel oder Lösungsmittelsystem vorgelegt ist, in einem Ultraschallbad befindet und das Reaktionsgemisch während der sukzessiven Zugabe der Verbindung der Formel **1** Ultraschall ausgesetzt wird. Anstelle der Verwendung eines Ultraschallbads kann in das Reaktionsgefäß, in welchem die Verbindung der Formel **2** in einem geeigneten Lösungsmittel oder Lösungsmittelsystem vorgelegt ist, eine Sonotrode (= Vorrichtung, welche die von einem Schallwandler erzeugten Ultraschallschwingungen an das zu beschallende Material weiterleitet) angebracht werden. Letztere Alternative bietet sich insbesondere für größere Ansätze an. Bezüglich Zugabegeschwindigkeit, Reaktionstemperatur und Lösungsmittel bzw. Lösungsmittelsystem müssen Vorversuche durchgeführt werden.

Alternativ oder zusätzlich erfolgt in einer bevorzugten Ausführungsform des erfindungsgemäßen Verfahrens die Durchführung unter Radiolysebedingungen. Hierbei werden solvatisierte Elektronen in wässriger Lösung durch Bestrahlung mit γ-Strahlung beispielsweise aus einer ⁶⁰Co-Quelle erzeugt. Diese Verfahrensweise wird näher in J.E. Packer et al., J. Chem. Soc., Perkin Trans. 2, 1975, 751 und im Aust. J. Chem. 1980, 33, 965 beschrieben, worauf hiermit in vollem Umfang Bezug genommen wird.

Der Begriff "Lösungsmittel" (Solvens, Lösemittel) bezeichnet im weitesten Sinne Stoffe, die andere auf physikalischem Wege ganz oder teilweise zur Lösung bringen können, im engeren Sinne anorganische und organische Flüssigkeiten, die andere gasförmige, flüssige oder feste Stoffe zu lösen vermögen. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Absorption in der Originalgestalt wiedergewonnen werden können.

Man kennt anorganische und organische Lösungsmittel, die hier gruppenweise vorgestellt werden.

Bei *anorganischen Lösungsmitteln* unterscheidet man Protonen-haltige (protische) wie z.B. H₂O, flüssiges NH₃, H₂S, HF, HCN, HNO₃, und Protonen-(Wasserstoff-)freie (aprotische)

Lösungsmittel, wie z.B. flüssiges SO₂, N₂O₄, NOCl, SeOCl₂, ICl, BrF₃, AsCl₃, zum anderen wässrige und nichtwässrige Lösungsmittel (alle außer H₂O, mit den Untergruppen aprotische, amphiprotische, protogene, protophile Lösungsmittel).

Die letzerwähnten Einteilungen lassen sich natürlich erst recht auf die organischen Lösungsmittel anwenden, von denen hier nur die wichtigsten aufgeführt sein können: Alkohole (z.B. Methanol, Ethanol usw.) Glykole (z. B. Ethylenglykol), Ether und Glykolether (z. B. Diethylether, Dioxan, Tetrahydrofuran), Ketone ( z.B. Aceton), Amide, Nitrile und andere Stickstoff-Verbindungen (z.B. Dimethylformamid, Acetonitril), Schwefelverbindungen (z. B. Schwefelkohlenstoff, Sulfolan), Nitroverbindungen (z. B. Nitromethan), Halogenkohlenwasserstoffe (z. B. Dichlormethan, Chloroform), Kohlenwasserstoffe (z. B. Benzine, Petrolether, Cyclohexan).

Geeignete Lösungsmittel oder Lösungsmittelsysteme hängen im einzelnen von der Wahl der jeweiligen Reaktionsbedingungen für die Zersetzung der Verbindung der Formel **1**, wie beispielsweise den Reaktionspartnern, ab. Es hat sich jedoch allgemein als vorteilhaft erwiesen, als Lösungsmittel oder Lösungsmittelsysteme für die Umsetzung der Verbindung der Formel **1** und der Verbindung der Formel **2** ein wässriges Lösungsmittel oder Lösungsmittelsystem zu verwenden. Vorzugsweise enthält das Lösungsmittel oder Lösungsmittelsystem Wasser.

Im Rahmen der vorliegenden Erfindung ist es nicht erforderlich, dass die Verbindungen der Formeln **1** und **2** vollständig im gewählten Lösungsmittel oder Lösungsmittelsystem löslich sind. Es hat sich umgekehrt sogar als vorteilhaft erwiesen, wenn speziell die Verbindung der Formel **2** im gewählten Lösungsmittel oder Lösungsmittelsystem nur teilweise löslich ist.

Im Rahmen der vorliegenden Erfindung sind bevorzugte organische Lösungsmittel beispielsweise kurzkettige Nitrile, wie Acetonitril oder Propionitril, Amide, wie Dimethylformamid, kurzkettige ein- oder mehrwertige Alkohole, wie Methanol, Ethanol, Propanol, Isopropanol, Ethylenglykol oder Trifluorethanol, kurzkettige Carbonsäuren, wie Eisessig, Trifluoressigsäure, kurzkettige Ketone, wie Aceton, und Dimethylsulfoxid oder Mischungen dieser organischen Lösungsmittel untereinander. Unter diesen ist Acetonitril bevorzugt.

Unter Säuren werden wässrige Lösungen von Mineralsäuren, wie Salzsäure, Bromwasserstoffsäure, Salpetersäure, Schwefelsäure, Phosphorsäure und dergleichen verstanden. Bevorzugt sind hierunter nichtoxidierende Säuren, wie Salzsäure oder Bromwasserstoffsäure.

Im Rahmen der erfindungsgemäßen Verfahren wird unter wässrigen Lösungsmitteln Wasser verstanden, dem vorzugsweise mindestens eine Base (z. B. Natriumhydroxid oder Kaliumhydroxid) zugesetzt sind.

Unter wässrigen Lösungsmittelsystemen wird eine Mischung aus Wasser mit wassermischbaren organischen und/oder anorganischen Lösungsmitteln verstanden, wobei wiederum bevorzugt eine oder mehrere Basen (z. B. Natriumhydroxid oder Kaliumhydroxid) zugesetzt sind. Wässrige Lösungsmittel und wässrige Lösungsmittelsysteme sind im Rahmen der vorliegenden Erfindung bevorzugt.

Im Rahmen der erfindungsgemäßen Verfahren sind geeignete Basen beispielsweise anorganische Basen, wie Alkalimetallhydroxide, z.B. Lithium-, Natrium- oder Kaliumhydroxid, Erdalkalimetallhydroxide, z.B. Magnesium- oder Calciumhydroxid, Aluminiumhydroxid, oder Alkali- und Erdalkalimetalloxide wie z.B. Natrium-, Magnesium- oder Calciumoxid.

Des Weiteren können Alkali- und Erdalkalicarbonate, z.B. Lithium-, Natrium-, Kalium- oder Calciumcarbonat, Alkali - und Erdalkalihydrogencarbonate, z.B. Lithium-, Natrium- oder Kaliumhydrogencarbonat, und organische Basen, wie Acetate, z.B. Natriumacetat, Alkoholate, z.B. Natriummethanolat, Natriumethanolat, Natrium-tert-butanolat oder Kalium-tert-butanolat und dergleichen; aliphatische und aromatische Amine, wie Diethylamin, Triethylamin, Ethyldiisopropylamin oder Pyridin als Basen eingesetzt werden. Die Alkoholate werden vorzugsweise als wässrige Lösungsmittelsysteme eingesetzt.

Bevorzugt sind die genannten anorganischen Basen, die vorzugsweise als wässrige Lösung verwendet werden, und insbesondere die genannten Alkali- und Erdalkalimetallhydroxide, wie Lithium-, Natrium- oder Kaliumhydroxid, vorzugsweise in Form ihrer wässrigen Lösung.

In einer bevorzugten Ausführungsform werden die Alkali- und Erdalkalimetallhydroxide dabei in verdünnter Form eingesetzt. "Verdünnt" bedeutet in diesem Zusammenhang, dass die Konzentration der Base 0,1 bis 50 Gew.-%, insbesondere 1 bis 32 Gew.-% und speziell 2 bis 16 Gew.-%, bezogen auf das Gesamtgewicht des Lösungsmittels beträgt.

Unter wässrigen Basen versteht man eine Lösung oder Dispersion der genannten Basen in Wasser.

Die wässrigen Lösungen der genannten anorganischen und/oder organischen Basen können auch im Gemisch mit den vorstehend genannten, wassermischbaren organischen Lösungsmitteln eingesetzt werden. In einer besonderen Ausführungsform wird die Konzentration der Base in dem wässrigen Lösungsmittel oder Lösungsmittelsystem so gewählt, dass der pH-Wert des Reaktionsgemisches größer als 9 ist (z.B. 9,1 oder größer), vorzugsweise 9,5 oder größer, insbesondere 10 oder größer (z.B. 9,1 bis 14; 9,5 bis 14 oder 10 bis 14).

Ebenso bevorzugt ist die Verwendung von Gemischen von mindestens zwei der genannten Basen, wenn dadurch der pH-Wert des Reaktionsgemisches größer als 9 ist (z.B. 9,1 oder größer), vorzugsweise 9,5 oder größer, insbesondere 10 oder größer (z.B. 9,1 bis 14; 9,5 bis 14 oder 10 bis 14).

Alternativ kann das erfindungsgemäße Verfahren aber auch nur in einem der genannten Lösungsmittel oder Lösungsmittelsysteme durchgeführt werden.

In einer bevorzugten Ausführungsform enthält das wässrige Lösungsmittel oder Lösungsmittelsystem eine Base, d.h. in dem wässrigen Lösungsmittel oder Lösungsmittelsystem liegt eine Base in einer Konzentration von in der Regel 0,1 bis 50 Gew.-%, insbesondere von 1 bis 32 Gew.-% und speziell von 2 bis 16 Gew.-% vor.

Als Basen werden hier vorzugsweise Natriumhydroxid oder Kaliumhydroxid verwendet.

Geeignet sind aber auch nichtwässrige Lösungsmittel oder Lösungsmittelsysteme, wie z.B. die o.g. organischen Lösungsmittel und Gemische dieser Lösungsmittel.

Bei Verwendung nichtwässriger Lösungsmittel oder Lösungsmittelsysteme besteht eine bevorzugten Ausführungsform wiederum darin, dass dem nichtwässrigen Lösungsmittel oder Lösungsmittelsystem mindestens eine der genannten Basen zugesetzt wird.

Unter "Lösungsmittelsystemen" wird eine Mischung aus mindestens zwei Lösungsmitteln verstanden, die unabhängig voneinander aus den Gruppen wässriger, organischer und/oder anorganischer Lösungsmittel ausgewählt sind. Bevorzugt ist Wasser eines der verwendeten Lösungsmittel des Lösungsmittelsystems.

Allgemein wird die Reaktion im "basischen Bereich" durchgeführt. Falls mindestens ein wässriges Lösungsmittel oder Lösungsmittelsystem verwendet wird, so zeigt das Reaktionsgemisch dabei einen pH-Wert von größer 9 (z.B. 9,1 oder größer), vorzugsweise 9,5 oder größer, insbesondere 10 oder größer (z.B. 9,1 bis 14; 9,5 bis 14 oder 10 bis 14). Der pH Wert kann dabei auch größer als 14 sein.

Falls mindestens ein nichtwässriges Lösungsmittel oder Lösungsmittelsystem verwendet wird, so bedeutet "im basischen Bereich", dass das betreffende Reaktionsgemisch bei Zusatz von Wasser einen pH-Wert von größer als 9 zeigen würde (z.B. 9,1 oder größer), vorzugsweise 9,5 oder größer, insbesondere 10 oder größer (z.B. 9,1 bis 14; 9,5 bis 14 oder 10 bis 14). Der pH Wert kann dabei auch größer als 14 sein.

Es können z. B. auch hochkonzentrierte Lösungen starker Basen verwendet werden, so dass der pH-Wert größer als 14 ist.

Ein weiteres geeignetes Lösungsmittelsystem ist ein Zweiphasen-Lösungsmittelsystem, welches zwei miteinander im Wesentlichen nicht mischbare Lösungsmittel oder Lösungsmittelsysteme umfasst. "Im Wesentlichen nicht mischbar" bedeutet, dass sich ein erstes Lösungsmittel oder Lösungsmittelsystem, das in geringerer oder gleicher Menge wie ein zweites Lösungsmittel oder Lösungsmittelsystem eingesetzt wird, im zweiten Lösungsmittel oder Lösungsmittelsystem zu höchstens 20 Gew.-%, vorzugsweise zu höchstens 10 Gew.-% und insbesondere zu höchstens 5 Gew.-%, bezogen auf das Gesamtgewicht des ersten Lösungsmittels oder Lösungsmittelsystems, löst. Beispiele sind Systeme, die neben einem wie oben definierten wässrigen Lösungsmittel bzw. Lösungsmittelsystem ein oder mehrere mit Wasser im Wesentlichen nicht mischbare Lösungsmittel enthalten, wie Carbonsäureester, z.B. Ethylacetat, Propylacetat oder Ethylpropionat, offenkettige Ether, wie Diethylether, Dipropylether, Dibutylether, Methylisobutylether und Methyl-tert-butylether, aliphatische Kohlenwasserstoffe, wie Pentan, Hexan, Heptan und Octan sowie Petrolether, halogenierte aliphatische Kohlenwasserstoffe, wie Methylenchlorid, Trichlormethan, Dichlorethan und Trichlorethan, cycloaliphatische Kohlenwasserstoffe, wie Cyclopentan und Cyclohexan, und aromatische Kohlenwasserstoffe, wie Benzol, Toluol, die Xylole, Chlorbenzol, Dichlorbenzole und Mesitylen.

Ein solches Zweiphasen-Lösungsmittelsystem kann außerdem wenigstens einen Phasentransferkatalysator enthalten.

Phasentransferkatalysatoren sind dem Fachmann hinreichend bekannt und umfassen beispielsweise geladene Systeme, wie organische Ammoniumsalze, beispielsweise Tetra-(C₁-C₁₈-alkyl)-ammoniumchloride oder -bromide, wie Tetramethylammoniumchlorid oder -bromid, Tetrabutylammoniumchlorid oder -bromid, Hexadecyltrimethylammoniumchlorid oder -bromid, Octadecyltrimethylammoniumchlorid oder -bromid, Methyltrihexylammoniumchlorid oder -bromid, Methyltrioctylammoniumchlorid oder - bromid oder Benzyltrimethylammoniumhydroxid (Triton B), ferner Tetra-(C₁-C₁₈-alkyl)-phosphoniumchloride oder -bromide, wie Tetraphenylphosphoniumchlorid oder -bromid, [(Phenyl)ₐ-(C₁-C₁₈-alkyl)_{b}]-phosphoniumchloride oder -bromide, worin a = 1 bis 3 und b = 3 bis 1 und die Summe a + b = 4 ist, und außerdem Pyridiniumsalze, wie Methylpyridiniumchlorid oder -bromid, und ungeladene Systeme, wie Kronenether oder Azakronenether, z.B. 12-Krone-4, 15-Krone-5, 18-Krone-6, Dibenzo-18-Krone-6 oder [2,2,2]-Kryptand (222-Kryptofix), Cyclodextrine, Calixarene, wie [14]-Metacyclophan, Calix[4]aren und p-tert-Butyl-Calix[4]aren, und Cyclophane.

Geeignet ist auch die Durchführung in einem Zweiphasensystem, wobei eine der Phasen wenigstens eines der oben genannten Lösungsmittel bzw. Lösungsmittelsysteme umfasst und die zweite Phase mit der ersten Phase im Wesentlichen nicht mischbar ist. Bevorzugt umfasst die erste Phase wenigstens eines der o.g. protischen Lösungsmittel, wie Wasser, Alkohole oder Diole. Besonders bevorzugt ist die erste Phase ein wässriges Lösungsmittel oder Lösungsmittelystem, dem mindestens eine Base, wie Natriumhydroxid, Kaliumhydroxid und dergleichen zugesetzt ist, oder ein Gemisch aus Wasser und mindestens einer Base mit wenigstens einem mit Wasser mischbaren organischen Lösungsmittel, wie z.B. Alkohole, wie Methanol, Ethanol, Propanol, Isopropanol oder Trifluorethanol, Diole, wie Ethylenglykol, cyclische Ether, wie Tetrahydrofuran und Dioxan, Acetonitril, Amide, wie Dimethylformamid, Carbonsäuren, wie Eisessig, und Ketone, wie Aceton. Insbesondere umfasst die erste Phase Wasser oder eine wässrige Lösung mindestens einer der genannten Basen, wobei die Base vorzugsweise Natriumhydroxid oder Kaliumhydroxid ist.

Die zweite Phase ist vorzugsweise ausgewählt unter offenkettigen Ethern, wie Diethylether, Dipropylether, Dibutylether, Methylisobutylether und Methyl-tert-butylether, aliphatischen Kohlenwasserstoffen, wie Pentan, Hexan, Heptan und Octan sowie Petrolether, halogenierten aliphatischen Kohlenwasserstoffen, wie Methylenchlorid, Trichlormethan, Tetrachlormethan, Dichlorethan und Trichlorethan, cycloaliphatischen Kohlenwasserstoffen, wie Cyclopentan und Cyclohexan, und aromatischen Kohlenwasserstoffen, wie Benzol, Toluol, die Xylole, Chlorbenzol oder Dichlorbenzol.

Im Rahmen der vorliegenden Erfindung versteht man unter nichtpolaren (apolaren) Lösungsmitteln beispielsweise Schwefelkohlenstoff, Tetrachlormethan, die meisten aromatischen und die gesättigten aliphatischen Kohlenwasserstoffe.

Der Begriff "schwach polare Lösungsmittel" bezeichnet beispielsweise Halogen- und manche aromatischen Kohlenwasserstoffe. Die empirische bestimmte und in Einheiten der sog. E_{T}(30)-Skala ausgedrückte Polarität steigt z.B. von apolarem n-Hexan über Toluol, Chloroform, n-Butanol, Aceton, Ethanol, Formamid bis zum stark polaren Wasser.

Im Rahmen der vorliegenden Erfindung versteht man unter "polaren organischen Lösungsmitteln" beispielsweise kurzkettige Nitrile, wie Acetonitril oder Propionitril, Amide, wie Dimethylformamid, kurzkettige ein- oder mehrwertige Alkohole, wie Methanol, Ethanol, Propanol, Isopropanol, Ethylenglykol oder Trifluorethanol, kurzkettige Carbonsäuren, wie Eisessig, Trifluoressigsäure, kurzkettige Ketone, wie Aceton, und Dimethylsulfoxid.

In einer alternativen Ausführungsform werden die Lösungsmittel oder Lösungsmittelsysteme in entgaster (d.h. speziell in von Sauerstoff befreiter) Form eingesetzt. Das Entgasen von Lösungsmitteln oder Lösungsmittelsystemen ist bekannt und kann beispielsweise durch ein- oder mehrfaches Einfrieren des Lösungsmittels oder Lösungsmittelsystems, Auftauen unter Vakuum (zum Entziehen des im Lösungsmittel oder Lösungsmittelsystem gelösten/dispergierten Gases) und Kompensieren mit einem Inertgas, wie Stickstoff oder Argon, erfolgen. Alternativ oder zusätzlich kann das Lösungsmittel oder Lösungsmittelsystem mit Ultraschall behandelt werden. Letztere Vorgehensweise bietet sich insbesondere bei Wasser bzw. wässrigen Lösungsmitteln oder Lösungsmittelsystemen an, da die Ausdehnung von Wasser beim Frieren zu apparativen Problemen führen kann.

Alternativ oder zusätzlich erfolgt in einer bevorzugten Ausführungsform des erfindungsgemäßen Verfahrens die Durchführung in wenigstens einem Lösungsmittel bzw. Lösungsmittelsystem, das die radikalische Zersetzung der Verbindung der Formel 1 in Stickstoff und ein Arylradikal bewirkt und/oder die Umsetzung in einer anderen Art und Weise begünstigt. "In einer anderen Art und Weise begünstigt" bedeutet beispielsweise, dass das Lösungsmittel bzw. Lösungsmittelsystem die Bildung des Arylradikals fördert.

Lösungsmittel bzw. Lösungsmittelsysteme, die die radikalische Zersetzung der Verbindung der Formel 1 in ein Arylradikal fördern, zeichnen sich durch ein gewisses reduktives Potential aus und können in Verbindung mit einer Verbindung der Formel 1 als Reduktionsmittel wirken; d.h. das Lösungsmittel oder mindestens ein Lösungsmittel des Lösungsmittelsystems ist selbst oxidierbar. Beispiele für solche Lösungsmittel sind Alkohole, z.B. C₁-C₄-Alkohole, wie Methanol, Ethanol, Propanol, Isopropanol, Butanol, sec-Butanol, Isobutanol und tert-Butanol, Diole, wie Ethylenglykol und Diethylenglykol, offenkettige Ether, wie Diethylether, Methylisobutylether und Methyl-tert-butylether, cyclische Ether, wie Tetrahydrofuran und Dioxan, stickstoffhaltige Heterocyclen, wie Pyridin, und HMPT (Hexamethylphosphorsäuretriamid), aber auch basische wässrige Lösungen. Geeignete Basen sind anorganische Basen, wie Alkalihydroxide, z.B. Lithium-, Natrium- oder Kaliumhydroxid, Erdalkalihydroxide, z.B. Magnesium- oder Calciumhydroxid, Aluminiumhydroxid, Alkali- und Erdalkalimetalloxide wie z.B. Natrium-, Magnesium- oder Calciumoxid, Alkalicarbonate, z.B. Lithium-, Natrium- oder Kaliumcarbonat, und Alkalihydrogencarbonate, z.B. Lithium-, Natrium- oder Kaliumhydrogencarbonat, und organische Basen, wie Acetate, z.B. Natriumacetat, oder Alkoholate, z.B. Natriummethanolat, Natriumethanolat, Natrium-tert-Butanolat oder Kalium-tert-Butanolat.

Geeignet sind besonders solche Lösungsmittel bzw. Lösungsmittelsysteme, die keine leicht abstrahierbaren Wasserstoffatome besitzen, da sie ein gebildetes Arylradikal bestmöglich vor Nebenreaktionen schützen.

Im Rahmen des erfindungsgemäßen Verfahrens ist es nicht notwendig, dass die Verbindung der Formel 2 im eingesetzten Lösungsmittel oder Lösungsmittelsystem vollständig löslich ist.

Beispiele für besonders geeignete Lösungsmittel bzw. Lösungsmittelsysteme sind Wasser im Gemisch mit mindestens einer Base, wie Natriumhydroxid, Kaliumhydroxid, Natriummethanolat, Natriumcarbonat, Kaliumcarbonat und dergleichen (insbesondere Natriumhydroxid, Kaliumhydroxid), aber auch Alkohole ohne Wasserstoffatome in der α-Position, wie tert-Butanol, oder vergleichsweise inerte organische Lösungsmittel bzw. Lösungsmittelsysteme, wie beispielsweise Acetonitril, Aceton, Trifluorethanol und/oder Dimethylsulfoxid. Insbesondere ein Zusatz von Wasser oder wässrigen Basen, wie Natriumhydroxid, Kaliumhydroxid, Natriummethanolat, Natriumcarbonat, Kaliumcarbonat und dergleichen, wirkt allgemein stabilisierend auf die entstehenden Arylradikale, da diese mit Wasser praktisch keine Nebenreaktionen eingehen. Wasser, dem mindestens eine Base zugesetzt wird, ist daher als Lösungsmittel ohne abstrahierbare Wasserstoffatome bevorzugt.

Wenn Lösungsmittel mit leicht abstrahierbaren Wasserstoffatomen, wie primäre Alkohole, eingesetzt werden, so werden sie vorzugsweise im Gemisch mit mindestens einem weiteren Lösungsmittel verwendet, das keine leicht abstrahierbaren Wasserstoffatome besitzt. Vorzugsweise werden die dem Arylradikal gegenüber nicht inerten organischen Lösungsmittel bzw. Lösungsmittel-systeme dabei in einer Menge von höchstens 50 Gew.-%, besonders bevorzugt von höchstens 20 Gew.-% und insbesondere von höchstens 10 Gew.-%, bezogen auf das Gewicht des Gemischs aus reduzierenden Lösungsmittel bzw. Lösungsmittelsystem und Lösungsmitteln bzw. Lösungsmittelsystemen ohne leicht abstrahierbare Wasserstoffatome, enthalten. Da als Lösungsmittel bzw. Lösungsmittelsysteme ohne leicht abstrahierbare Wasserstoffatome insbesondere Wasser oder wässrige Basen eingesetzt werden, sind die in den Gemischen eingesetzten reduzierenden Lösungsmittel bzw. Lösungsmittelsysteme vorzugsweise solche, die mit Wasser mischbar sind (das sind die zuvor genannten Alkohole, Diole und cyclischen Ether).

Insgesamt werden als Lösungsmittel bzw. Lösungsmittelsysteme, die die radikalische Zersetzung der Verbindung der Formel **1** in Stickstoff und ein Arylradikal bewirken und/oder die Umsetzung in einer anderen Art und Weise begünstigen, vorzugsweise Wasser, die genannten wässrigen Basen oder Gemische der oben genannten organischen, mit Wasser mischbaren, ein gewisses Reduktionspotential aufweisenden Lösungsmittel bzw. Lösungsmittelsysteme (das sind die zuvor genannten Alkohole, Diole und cyclischen Ether) mit Wasser oder den genannten wässrigen Basen eingesetzt.

Alternativ bevorzugt ist die Durchführung in (alleiniger) Gegenwart wenigstens eines Lösungsmittels, das die radikalische Zersetzung der Verbindung der Formel **1** in Stickstoff und ein Arylradikal bewirkt und/oder die Umsetzung in einer anderen Art und Weise begünstigt, insbesondere von Wasser, mindestens einer der oben genannten wässrigen Basen oder eines Gemisches der genannten reduktiv wirkenden, organischen, mit Wasser mischbaren Lösungsmittel mit Wasser oder mindestens einer der oben genannten wässrigen Basen. Die Lösungsmittel oder Lösungsmittelsysteme können in entgaster oder nicht entgaster Form eingesetzt werden.

Alternativ verwendet man nicht die reinen Lösungsmittel, sondern Gemische, die die Lösungseigenschaften vereinigen, oder man greift zu Lösungsvermittlern.

Der Begriff "Lösungsvermittler" bezeichnet (grenzflächenaktive) Stoffe, die durch ihre Gegenwart andere, in einem Lösungsmittel oder Lösungsmittelsystem praktisch unlösliche Verbindungen in diesem Lösungsmittel oder Lösungsmittelsystem löslich oder emulgierbar machen. Es gibt Lösungsvermittler, die mit der schwer löslichen Substanz eine Molekülverbindung eingehen und solche, die durch Micellen-Bildung wirken. Man kann auch sagen , dass Lösungsvermittler einem sog. latenten Lösungsmittel sein Lösungsvermögen verleihen. Beispielsweise ist Ethanol (als latentes Lösungsmittel) löst Celluloseacetat schlecht, nach Zusatz von 2-Nitropropan (als Lösungsvermittler) jedoch viel besser.

Die erfindungsgemäße Umsetzung erfolgt, indem man die Verbindung der Formel **1** und eine Verbindung der Formel **2** vorzugsweise in einem geeigneten Lösungsmittel oder Lösungsmittelsystem miteinander unter Reaktionsbedingungen in Kontakt bringt, die eine Zersetzung der Verbindung der Formel **1** in Stickstoff und ein Arylradikal bewirken.

Die Reaktanden können prinzipiell in unterschiedlicher Reihenfolge miteinander in Kontakt gebracht werden. So kann beispielsweise die Verbindung der Formel **2**, gegebenenfalls in einem Lösungsmittel bzw. Lösungsmittelsystem gelöst oder dispergiert, vorgelegt und mit der Verbindung der Formel **1**, gegebenenfalls in einem Lösungsmittel bzw. Lösungsmittelsystem gelöst oder dispergiert, versetzt werden.

Umgekehrt kann die Verbindung der Formel **1**, gegebenenfalls in einem Lösungsmittel bzw. Lösungsmittelsystem gelöst oder dispergiert, vorgelegt und mit der Verbindung der Formel **2**, gegebenenfalls in einem Lösungsmittel bzw. Lösungsmittelsystem gelöst oder dispergiert, versetzt werden.

Es hat sich jedoch als vorteilhaft erwiesen, die Verbindung der Formel **2** gegebenenfalls in einem Lösungsmittel bzw. Lösungsmittelsystem vorzulegen und die Verbindung der Formel **1**, gegebenenfalls in einem Lösungsmittel bzw. Lösungsmittelsystem gelöst oder dispergiert, hinzuzugeben.

In einer alternativen Ausführungsform können die Verbindungen der Formel **1** und der Formel **2** zunächst in einem ersten Lösungsmittel oder Lösungsmittelsystem gelöst oder dispergiert werden, und dann ein zweites Lösungsmittel oder Lösungsmittelsystem zugesetzt werden, das die Zersetzung der Verbindung der Formel **1** in Stickstoff und ein Arylradikal bewirkt.

Vorteilhaft ist auch die Zugabe des Gemisches aus den Verbindungen der Formeln **1** und **2** und dem ersten Lösungsmittel oder Lösungsmittelsystem zum zweiten Lösungsmittel oder Lösungsmittelsystem.

Das erste Lösungsmittel oder Lösungsmittelsystem wird dabei bevorzugt so gewählt, dass noch keine Zersetzung der Verbindung der Formel **1** in Stickstoff und Arylradikal erfolgt. Speziell sind zu diesem Zweck Lösungsmittel oder Lösungsmittelsysteme geeignet, die durch Anwesenheit mindestens einer Säure einen pH-Wert von höchstens 7 aufweisen.

Das zweite Lösungsmittel oder Lösungsmittelsystem wird dabei bevorzugt so gewählt, dass es nach der Vereinigung mit dem Gemisch der Verbindung der Formel **1** und der Verbindung der Formel **2**, die im ersten Lösungsmittel oder Lösungsmittelsystem gelöst oder dispergiert sind, die Zersetzung der Verbindung der Formel **1** in Stickstoff und ein Arylradikal bewirkt. Bevorzugt enthält das zweite Lösungsmittel oder Lösungsmittelsystem mindestens eine Base. Die Base sollte gegenüber der genannten Säure (Zusatz zum ersten Lösungsmittel oder Lösungsmittelsystem) im Überschuss vorliegen, so dass nach Zugabe aller Komponenten die Reaktion im basischen Bereich abläuft.

Die Umsetzung kann sowohl in einem Lösungsmittel bzw. Lösungsmittelsystem als auch in Substanz durchgeführt werden. In letzterem Fall fungiert beispielsweise die Verbindung der Formel **2** selbst als Lösungs- oder Dispergiermittel oder wird, falls sein Schmelzpunkt oberhalb Raumtemperatur (25°C) liegt, als Schmelze vorgelegt und dann mit der Verbindung der Formel **1** unter geeigneten Reaktionsbedingungen versetzt. Die bevorzugte Ausführungsform ist jedoch die Durchführung in einem Lösungsmittel bzw. Lösungsmittelsystem, das gegebenenfalls mindestens eine Base enthält.

Vorzugsweise wird die Verbindung der Formel **2** direkt als freies Amin eingesetzt. Alternativ kann es auch, entweder vollständig oder teilweise, in Form eines seiner Säureaddukte oder einer Mischung solcher Addukte eingesetzt werden, wobei das Hydrochlorid der Verbindung der Formel **2** besonders bevorzugt ist. Bei der Verwendung der Säureaddukte der Verbindung der Formel **2** muss durch Zusatz mindestens einer Base gewährleistet sein, dass die Reaktion (d.h. die Zersetzung der Verbindung der Formel **1** in Stickstoff und ein Arylradikal) wiederum im basischen Bereich abläuft.

Die Durchführung erfolgt beispielsweise so, dass die Verbindung der Formel **2** in einem Lösungsmittel oder Lösungsmittelsystem vorgelegt und dann die Verbindung der Formel **1** sukzessive zugegeben wird oder umgekehrt die Verbindung der Formel 1 in einem solchen Lösungsmittel oder Lösungsmittelsystem vorgelegt und dann die Verbindung der Formel **2** zugegeben wird, wobei die erste Variante bevorzugt ist. Bezüglich Zugabegeschwindigkeit und Reaktionstemperatur wird auf die folgenden Ausführungen verwiesen. Bei Durchführung in einem Zweiphasensystem kann alternativ die Verbindung der Formel 2 im Lösungsmittel oder Lösungsmittelsystem der einen Phase und die Verbindung der Formel 1 im Lösungsmittel oder Lösungsmittelsystem der zweiten Phase jeweils vorgelegt werden.

Wie bereits gesagt, wird in einer bevorzugten Ausführungsform die Verbindung der Formel 1, zu der in der Vorlage befindlichen Verbindung der Formel 2 gegeben. Die Verbindung der Formel 1 kann sowohl in Substanz als auch in einem Lösungsmittel oder Lösungsmittelsystem gelöst oder dispergiert zugegeben werden. Als Lösungsmittel können hier Wasser oder die oben genannten wässrigen Lösungsmittelsysteme oder polare organische Lösungsmittel eingesetzt werden, wobei gegebenenfalls mindestens eine Base zugesetzt werden kann. Die Verbindung der Formel **1** wird dabei vorzugsweise sukzessive (portionsweise oder kontinuierlich) zugegeben. Die sukzessive Zugabe unterdrückt in vielen Fällen die Bildung von Homokupplungsprodukten, d.h. von Produkten, die durch Reaktion von zwei oder mehreren Verbindungen der Formel **1** miteinander entstehen, denn eine geringe Konzentration der Verbindung der Formel **1** im Reaktionsgemisch gewährleistet, dass seine Umsetzung mit der Verbindung der Formel **2** gegenüber der Umsetzung mit sich selbst überwiegt.

Die Geschwindigkeit der Zugabe wird dabei von mehreren Faktoren bestimmt, wie Ansatzgröße, Temperatur, Reaktivität der Edukte und Art der gewählten Reaktionsbedingungen, die eine Zersetzung der Verbindung der Formel **1** in Stickstoff und ein Arylradikal bewirkt, und kann vom Fachmann im Einzelfall, beispielsweise durch geeignete Vorversuche, bestimmt werden. So verlangt eine geringe Reaktivität der Edukte eine langsamere Zugabegeschwindigkeit, die aber beispielsweise durch eine höhere Temperatur und/oder durch die Wahl von Reaktionsbedingungen, die eine Zersetzung der Verbindung der Formel **1** beschleunigen, zumindest teilweise kompensiert werden kann.

Die beiden bevorzugten Maßnahmen, d.h. der Einsatz der Verbindung der Formel **1** im Unterschuss (bzgl. der Verbindung der Formel **2**) sowie deren schrittweise Zugabe, bewirken einen vorteilhaften Reaktionsverlauf, da sie die Homokupplung der Verbindung der Formel **1** zurückdrängen.

Erfindungsgemäß wird die Verbindung der Formel **1** und die Verbindung der Formel **2** unter Reaktionsbedingung umgesetzt, die eine Zersetzung der Verbindung der Formel **1** in Stickstoff und ein Arylradikal bewirken. Dabei werden bevorzugt Reaktionsbedingungen gewählt, unter denen auf die Verbindung der Formel **1** ein einzelnes Elektron (SET; single electron transfer) übertragen wird.

Geeignete Bedingungen, unter denen eine Zersetzung der Verbindung der Formel **1** in Stickstoff und ein Arylradikal stattfindet, sind dem Fachmann allgemein bekannt. So kann bereits die Zugabe der Verbindung der Formel **1** zu der Verbindung der Formel **2** die Zersetzung der Verbindung der Formel **1** in Stickstoff und einem Arylradikal bewirken, da zumindest ein Teil der Verbindung der Formel **2** ein ausreichendes reduktives Potential besitzt. In diesem Fall müssen keine speziellen Verfahrensmaßnahmen ergriffen werden und die Reaktion kann unter den oben beschriebenen Reaktionsbedingungen durchgeführt werden. Reicht das Reduktionspotential der Verbindung der Formel **2** nicht aus, ist es erforderlich, weitere Verfahrensmaßnahmen zu ergreifen, um den Reduktionsschritt einzuleiten. Aber auch wenn das Reduktionspotential des eingesetzten Anilins ausreichen sollte, um die Zersetzung der Verbindung der Formel **1** in Stickstoff und ein Arylradikal einzuleiten, kann es von Vorteil sein, weitere Verfahrensmaßnahmen zu ergreifen, die einen Zerfall oder Abbau der Verbindung der Formel **1** in Stickstoff und ein Arylradikal gewährleisten/beschleunigen bzw. gegenüber anderen Reaktionsmöglichkeiten der Verbindung der Formel **1** (z.B. Azokupplung) bevorzugt ablaufen lassen.

Werden zwei oder mehrere der obigen Maßnahmen kombiniert, so ist vorzugsweise eine dieser Maßnahmen die Durchführung in Gegenwart wenigstens eines Lösungsmittels oder Lösungsmittelsystems und/oder die Durchführung in Gegenwart wenigstens eines Lösungsmittels bzw. Lösungsmittelsystems, das die radikalische Zersetzung der Verbindung der Formel 1 in Stickstoff und ein Arylradikal bewirkt und/oder die Umsetzung in einer anderen Art und Weise begünstigt. Geeignet ist insbesondere die Kombination dieser beiden Maßnahmen untereinander sowie mit der Durchführung unter Zusatz mindestens einer Base. Die Reaktionstemperatur wird von mehreren Faktoren bestimmt, wie beispielsweise der Reaktivität der eingesetzten Edukte und der Art der gewählten Reaktionsbedingung, die eine Zersetzung der Verbindung der Formel **1** in Stickstoff und ein Arylradikal bewirkt, und kann vom Fachmann im Einzelfall ermittelt werden, beispielsweise durch einfache Vorversuche. Im Allgemeinen führt man die Umsetzung der Verbindung der Formel **1** mit der Verbindung der Formel **2** bei einer Temperatur im Bereich von vorzugsweise -100 °C bis zum Siedepunkt des Reaktionsgemischs, bevorzugt -78 °C bis 200 °C, besonders bevorzugt von 0 °C bis 150 °C und insbesondere von 50 °C bis 130 °C (z.B. 70 bis 110 °C) durch. Diese Temperaturen gelten für die Durchführung in Lösung; wird der Versuch hingegen in Substanz durchgeführt und liegt der Schmelzpunkt der Verbindung der Formel **2** über Raumtemperatur, so entspricht die Reaktionstemperatur selbstverständlich mindestens der Temperatur der Schmelze des Reaktionsgemischs.

Vorzugsweise setzt man in dem erfindungsgemäßen Verfahren zur Herstellung von Verbindungen der Formel 3 die Verbindung der Formel 1 in einer Menge von 0,001 bis 5,0 Mol, besonders bevorzugt von 0,002 bis 1,0 Mol (z. B. 0,02 bis 0,5 Mol) und insbesondere von 0,05 bis 0,3 Mol (z.B. 0,05 bis 0,2 Mol), jeweils bezogen auf 1 Mol der Verbindung der Formel 2 ein.

Verbindungen der Formel 1 sind allgemein bekannt und können gemäß gängiger Verfahren hergestellt werden, wie sie beispielsweise in Organikum, Wiley VCH, 22. Auflage beschrieben sind. So sind sie durch Diazotierung des entsprechenden Anilinderivates erhältlich, beispielsweise, indem man ein solches Anilinderivat mit Nitrit in Gegenwart einer Säure, wie etwa halbkonzentrierte Schwefelsäure, umsetzt. Sowohl entsprechende Anilinderivate zur Herstellung von Verbindungen der Formel 1 als auch von Verbindungen der Formel 2 sind bekannt oder können nach bekannten Verfahren hergestellt werden, beispielsweise, indem man entsprechend substituierte Nitrobenzole in Gegenwart eines geeigneten Katalysators hydriert oder homogen reduziert (etwa mit Sn(II)-Chlorid / HCl, vgl. Houben Weyl, "Methoden d. org. Chemie" 11/1, 422). Auch die Herstellung aus Azobenzolen und die Substitution geeigneter Benzole mit Ammoniak sind gängige Methoden. Die Herstellung von Verbindungen der Formel 1 bei denen die Gegenanionen unter den Anionen von aromatischen Dicarbonsäureimiden oder Disulfonimiden ausgewählt sind, kann analog zu M. Barbero et al., Synthesis 1998, 1171-1175 erfolgen.

Die Aufarbeitung der erhaltenen Reaktionsgemische und die Isolierung der Verbindungen der Formel 3 erfolgt in üblicher Weise, beispielsweise durch eine extraktive Aufarbeitung, durch Entfernen des Lösungsmittels, z. B. unter vermindertem Druck, oder durch eine Kombination dieser Maßnahmen. Eine weitere Reinigung kann beispielsweise durch Kristallisation, Destillation oder durch Chromatographie erfolgen.

Überschüssige bzw. nicht umgesetzte Edukte (hierbei handelt es sich vor allem um die Verbindung der Formel 2, die in Bezug auf die Verbindung der Formel 1 bevorzugt im Überschuss eingesetzt wird) werden bei der Aufarbeitung vorzugsweise isoliert und erneut verwendet.

Entsprechend einer bevorzugten Ausführungsform der Erfindung wird das Reaktionsgemisch zur Aufarbeitung mit Wasser verdünnt und mit einem geeigneten, im Wesentlichen nicht wassermischbaren organischen Lösungsmittel mehrmals extrahiert und die kombinierten organischen Phasen eingeengt. Je nach den Säure-Base-Eigenschaften des Produktes wird der pH-Wert vor der Extraktion gegebenenfalls durch Zugabe von Säuren oder Basen geeignet eingestellt. Beispiele für geeignete, im Wesentlichen nicht wassermischbare organische Lösungsmittel sind oben aufgeführt. Das so isolierte Produkt kann anschließend für Verwendungen bereitgehalten oder direkt einer Verwendung zugeführt werden, beispielsweise in einem weiteren Reaktionsschritt eingesetzt werden, oder zuvor weiter aufgereinigt werden.

Beispiele:

### Herstellung von Aryldiazoniumchloriden mit Natriumnitrit (AAV 1)

Zu einer im Eisbad gekühlten und mit Stickstoff entgasten Lösung des Anilinderivats (20.0 mmol) in Salzsäure (3 N, 20 mL) und Wasser (20 mL) wird über 10 Minuten eine mit Stickstoff entgaste Lösung aus Natriumnitrit (20.0 mmol, 1.38 g) in Wasser (10 mL) zugetropft. Im Eisbad wird für weitere 20 Minuten gerührt, die klare Lösung kann anschließend für weitere Reaktionen verwendet werden. Die Konzentration der Aryldiazoniumchlorid-Lösung beträgt 0.4 M (20.0 mmol / 50 mL).

### Herstellung von Aryldiazoniumtetrafluoroboraten mit Natriumnitrit (AAV 2)

Ein Gemisch aus dem jeweiligen Anilinderivat (40.0 mmol), Tetrafluorborsäure (50%, 80.0 mmol, 14.0 mL) und Wasser (15 mL) werden im Eisbad auf 0-5 °C gekühlt. Es wird langsam eine vorgekühlte Lösung aus Natriumnitrit (42.0 mmol, 2.90 g) in Wasser (6.5 mL) zugetropft, sodass die Temperatur stets unter 5 °C bleibt. Nach 30-minütigem Rühren bei unveränderter Temperatur wird das Diazoniumsalz abfiltriert und mit kaltem Diethylether gewaschen. Lösungsmittelreste werden bei Raumtemperatur im Vakuum entfernt. Die Ausbeuten betragen zwischen 80% und 95%. Die so erhaltenen Aryldiazoniumtetrafluoroborate können für mehrere Wochen bei unter -18 °C gelagert werden.

### Allgemeine Vorschrift zur Biarylsynthese (AAV 3)

Unter starkem Rühren wird zu einem auf 70 °C erwärmten Anilinderivat (25.0 mmol) eine Suspension aus einem Aliquot (2.00 mmol, 5.00 mL) der 0.4 M Diazoniumchloridlösung aus AAV 1 und wässriger Natriumhydroxidlösung (4 N, 3 mL) über einen Zeitraum von 10-15 Minuten zugetropft. Alternativ kann zur Herstellung der Suspension auch eine Lösung des Diazoniumtetrafluoroborates (2.00 mmol, aus AAV 2) in einem Gemisch aus Wasser und Acetonitril (3 mL + 2 mL) verwendet werden.

Nach erfolgter Zugabe wird für weitere 10 Minuten gerührt und anschließend das Reaktionsgemisch mit gängigen organischen Lösungsmitteln (z. B. Diethylether, Dichlormethan oder Ethylacetat) extrahiert (3 x 75 mL). Die vereinten organischen Phasen werden mit gesättigter, wässriger Natriumchloridlösung gewaschen und über Natriumsulfat getrocknet. Das Lösungsmittel wird bei vermindertem Druck entfernt und das erhaltene Produkt im Vakuum getrocknet. Die weitere Aufreinigung der Produkte erfolgt, je nach Substanz, mittels Vakuumdestillation, Kugelrohrdestillation oder Säulenchromatographie an Kieselgel.

Die Ausbeute der Biphenylsynthese bezieht sich auf das Gemisch von Diazotat, Diazohydroxid und Diazoanhydrid, das aus der Zugabe der wässrigen Natriumhydroxidlösung zur Diazoniumchloridlösung als Suspension erhalten wird. Der Anteil der Verbindungen, aus denen im Folgeschritt Arylradikale gebildet werden können, wird mittels dreier unabhängiger Methoden (NMR, Stickstoffentwicklung, Photometrie) zu 75% bezogen auf das in AAV 1 eingesetzte Anilinderivat bestimmt. Wird eine Suspension aus gelöstem Aryldiazoniumtetrafluoroborat und wässriger Natriumhydroxidlösung verwendet, so bezieht sich der 75%-Gehalt der Verbindungen, die Arylradikale bilden können, auf die Menge an eingewogenem Aryldiazoniumtetrafluoroborat.

### Allgemeine Vorschrift zur Biarylsynthese (AAV 4)

Unter starkem Rühren wird zu einer auf 70 °C erwärmten Suspension aus dem Anilinderivat (25.0 mmol) und wässriger Natriumhydroxidlösung (4 N, 3 mL) ein Aliquot (2.00 mmol, 5.00 mL) der 0.4 M Diazoniumchloridlösung aus AAV 1 über einen Zeitraum von 10-15 Minuten zugetropft. Nach erfolgter Zugabe wird für weitere 10 Minuten gerührt und anschließend das Reaktionsgemisch mit gängigen organischen Lösungsmitteln (z. B. Diethylether, Dichlormethan oder Ethylacetat) extrahiert (3 × 75 mL). Die vereinten organischen Phasen werden mit gesättigter, wässriger Natriumchloridlösung gewaschen und über Natriumsulfat getrocknet. Das Lösungsmittel wird bei vermindertem Druck entfernt und das erhaltene Produkt im Vakuum getrocknet. Die weitere Aufreinigung der Produkte erfolgt, je nach Substanz, mittels Vakuumdestillation, Kugelrohrdestillation oder Säulenchromatographie an Kieselgel.

4'-Chlor-5- fluorbiphenyl-2-amin

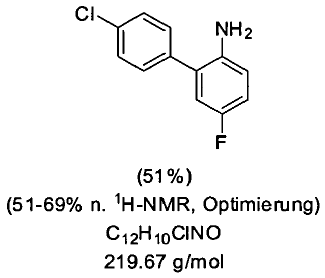

Zur Ermittlung der als AAV3 beschriebenen Reaktionsbedingungen wurden folgende Optimierungsversuche durchgeführt.

4'-Chlor-5-fluorbiphenyl-2-amin wird aus 4-Fluoranilin (25.0 mmol, 2.40 mL) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) entsprechend der allgemeinen Arbeitsvorschrift AAV 3 und den in Tabelle 1 angegebenen Variationen dieser Vorschrift synthetisiert. Es wird mit Diethylether extrahiert und im Vakuum eingeengt.

Die in Tabelle 1 angegebenen Ausbeuten wurden mittels ¹H-NMR-Spektrometrie (interner Standard: Diethylterephthalat) bestimmt und beziehen sich auf einen 75%igen Gehalt der zwischenzeitlich erhaltenen Suspension (siehe oben).

| Reaktionsbedingungen | Ausbeute an 4'-Chlor-5-fluorbiphenyl-2-amin [%] (mittels ¹H-NMR und int. Standard Dimethylterephthalat) |
|---|---|
| Standardbedingungen, siehe AAV 3 | 63 |
| Reaktionsführung bei 50 °C | 61 |
| Reaktionsführung bei 90 °C | 68 |
| Reaktionsführung bei 110 °C | 64 |
| Reaktion unter Stickstoffatmosphäre | 69 |
| Reaktion unter Argonatmosphäre | 64 |
| Zugabezeit des Diazoniumsalzes: 6 min | 51 |
| Zugabezeit des Diazoniumsalzes: 24 min | 61 |
| 8 N Natriumhydroxidlösung | 67 |
| 4-Fluoranilin (20.0 mmol) | 68 |
| nach AAV 4 | 52 (bzgl. 4-Chloranilin) |

Im präparativen Versuch wird 4'-Chlor-5-fluorbiphenyl-2-amin aus 4-Fluoranilin (25.0 mmol, 2.40 mL) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Der Überschuss an 4-Fluoranilin wird durch Vakuumdestillation entfernt und das erhaltene Rohprodukt säulenchromatographisch (Kieselgel, Hexan / EtOAc = 4:1) gereinigt. Es wird 4'-Chlor-5-fluorbiphenyl-2-amin (0.76 mmol, 167 mg, 51%) erhalten.
- *R*_{f}-Wert: 0.4 (Hexan / EtOAc = 4:1) [UV]
- ¹H-NMR: (360 MHz, CDCl₃): δ = 3.58 (s, 2 H), 6.69 (dd, *J*_{HF} = 4.8 Hz, *J* = 8.6 Hz, 1 H), 6.83 (dd, *J* = 3.0 Hz, J_{HF} = 9.2 Hz, 1 H), 6.88 (ddd, *J* = 3.0 Hz, *J*_{HF}= 8.2 Hz, *J* = 8.6 Hz, 1 H), 7.38 (d, *J* = 8.7 Hz, 2 H), 7.43 (d, *J* = 8.7 Hz, 2 H).
- ¹³C-NMR: (90.6 MHz, CDCl₃)_{:} δ = 115.2 (d, *J*_{CF} = 22.2 Hz, CH), 116.5 (d, *J*_{CF} = 22.6 Hz, CH), 116.6 (d, *J*_{CF} = 7.7 Hz, CH), 127.2 (d, *J*_{CF} = 7.1 Hz, C_{q}), 129.1 (2xCH), 130.3 (2xCH), 133.6 (Cq), 136.9 (d, *J*_{CF} = 1.7 Hz, Cq), 139.5 (d, *J*_{CF} = 2.1 Hz, C_{q}), 156.3 (d, *J*_{CF} = 236.7 Hz, Cq).

4'-Chlor-5-methoxybiphenyl-2-amin und 4'-Chlor-6-methoxybiphenyl-3-amin

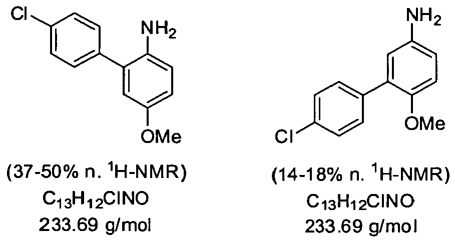

4'-Chlor-5-methoxybiphenyl-2-amin und 4'-Chlor-6-methoxybiphenyl-3-amin werden aus *p-*Anisidin (20.0 mmol, 2.46 g) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert und im Vakuum eingeengt. Die Ausbeuten der beiden Regioisomere 4'-Chlor-5-methoxybiphenyl-2-amin (0.56-0.75 mmol, 131-175 mg, 37-50%) und 4'-Chlor-6-methoxybiphenyl-3-amin (0.21-0.27 mmol, 49-63 mg, 14-18%) werden mittels ¹H-NMR-Spektrometrie bestimmt (interner Standard: Dimethylterephthalat).

### 4'-Chlor-5-methoxybiphenyl-2-amin:

- *R*_{f}-Wert: 0.7 (CH₂Cl₂ / EtOAc = 20:1) [UV]
- ¹H-NMR: (360 MHz, CDCl₃): δ = 3.77 (s, 3 H), 3.90 (s, 2 H), 6.70 (d, *J* = 2.7 Hz, 1 H), 6.76 (dd, *J* = 0.6 Hz, *J* = 8.6 Hz, 1 H), 6.79 (dd, *J* = 2.7 Hz, *J* = 8.6 Hz, 1 H), 7.41 (s, 4 H).
- ¹³C-NMR: (90.6 MHz, CDCl₃): δ = 55.8 (CH₃), 114.7 (CH), 115.7 (CH), 117.4 (CH), 127.9 (Cq), 128.9 (2×CH), 130.4 (2×CH), 133.3 (Cq), 136.3 (Cq), 137.7 (Cq), 153.1 (Cq).

### 4'-Chlor-6-methoxybiphenyl-3-amin:

- *R*_{f}-Wert: 0.5 (CH₂Cl₂ / EtOAc = 20:1) [UV]
- ¹H-NMR: (360 MHz, CDCl₃): δ = 3.44 (s, 2 H), 3.72 (s, 3 H), 6.66-6.69 (m, 2 H), 6.81-6.84 (m, 1 H), 7.36 (d, *J* = 8.8 Hz, 2 H), 7.46 (d, *J* = 8.8 Hz, 2 H).
- ¹³C-NMR: (90.6 MHz, CDCl₃): δ = 56.3 (CH₃), 113.2 (CH), 115.3(CH), 117.9 (CH), 128.1 (2×CH), 130.3 (Cq), 130.7 (2×CH), 132.8 (Cq), 136.9 (Cq), 140.2 (Cq), 149.6 (Cq).

4',5-Dichlorbiphenyl-2-amin

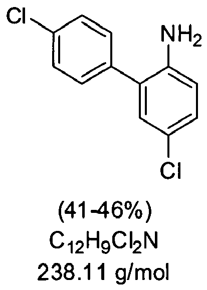

4',5-Dichlorbiphenyl-2-amin wird aus 4-Chloranilin (20.0 mmol, 2.54 g) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Das erhaltene Rohprodukt wird mittels Säulenchromatographie (Kieselgel, Hexan / EtOAc = 4:1) gereinigt, wodurch 4',5-Dichlorbiphenyl-2-amin (0.61-0.69 mmol, 145-164 mg, 41-46%) erhalten wird.
- *R*_{f}-Wert: 0.5 (Hexan / EtOAc = 4:1) [UV]
- ¹H-NMR: (360 MHz, CDCl₃): δ = 6.68 (d, *J* = 8.5 Hz, 1 H), 7.06 (d, *J* = 2.5 Hz, 1 H), 7.11 (dd, *J* = 2.5 Hz, *J=* 8.5 Hz, 1 H), 7.35-7.44 (m, 4 H).
- ¹³C-NMR: (90.6 MHz, CDCl₃): δ = 116.9 (CH), 123.4 (Cq), 128.5 (CH), 129.2 (2xCH), 129.8 (CH), 130.3 (2xCH), 131.5 (Cq), 133.7 (Cq), 136.7 (Cq), 141.9 (Cq).

4',5-Difluorbiphenyl-2-amin

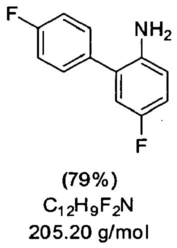

4',5-Difluorbiphenyl-2-amin wird aus 4-Fluoranilin (25.0 mmol, 2.40 mL) und 4-Fluorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Der Überschuss an 4-Fluoranilin wird durch Vakuumdestillation entfernt und das erhaltene Rohprodukt säulenchromatographisch (Kieselgel, Hexan / EtOAc = 4:1) gereinigt. Es wird 4',5-Difluorbiphenyl-2-amin (1.18 mmol, 242 mg, 79%) erhalten.
- *R*_{f}-Wert: 0.4 (Hexan / EtOAc = 4:1) [UV]

- ¹H-NMR: (360 MHz, CDCl₃): δ = 6.69 (ddd, *J* = 0.4 Hz, J_{HF} = 4.9 Hz, *J* = 8.7 Hz, 1 H), 6.81-6.90 (m, 2 H), 7.13 (t, *J* = 8.7 Hz, *J*_{HF} = 8.7 Hz, 2 H), 7.40 (dd, *J*_{HF} = 5.4 Hz, J = 8.7 Hz, 2 H).
- ¹³C-NMR: (90.6 MHz, CDCl₃): δ = 115.0 (d, *J*_{CF} = 22.2 Hz, CH), 115.9 (d, *J*_{CF} = 21.4 Hz, 2xCH), 116.6 (d, J_{CF} = 7.2 Hz, CH), 116.7 (d, J_{CF} = 23.1 Hz, CH), 127.7 (d, *J*_{CF} = 7.2 Hz, C_{q}), 130.6 (d, *J*_{CF} = 8.0 Hz, 2xCH), 134.4 (dd, *J*_{CF} = 1.7 Hz, J_{CF} = 3.4 Hz, C_{q}), 139.5 (d, *J*_{CF} = 2.3 Hz, C_{q}), 156.3 (d, *J*_{CF} = 236.6 Hz, C_{q}), 162.2 *(*d, J_{CF} = 247.1 Hz, Cq).

5-Fluorbiphenyl-2-amin

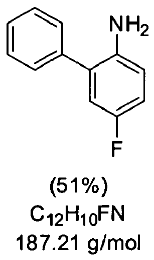

5-Fluorbiphenyl-2-amin wird aus 4-Fluoranilin (25.0 mmol, 2.40 mL) und einer Suspension aus Phenyldiazoniumtetrafluoroborat (2.00 mmol, 384 mg; hergestellt nach der allgemeinen Arbeitsvorschrift AAV 2), Acetonitril (4 mL) und wässriger Natriumhydroxidlösung (4 N, 3 mL) analog der allgemeinen Arbeitsvorschrift AAV 3 synthetisiert. Es wird mit Diethylether extrahiert. Der Überschuss an 4-Fluoranilin wird durch Vakuumdestillation entfernt und das erhaltene Rohprodukt säulenchromatographisch (Kieselgel, Hexan / EtOAc = 4:1) gereinigt. Es wird 5-Fluorbiphenyl-2-amin (0.76 mmol, 142 mg, 51 %) erhalten.
- *R*_{f}-Wert: 0.3 (Hexan / EtOAc = 4:1) [UV]
- ¹H-NMR: (360 MHz, CDCl₃): δ = 6.69 (dd, *J*_{HF} = 4.8 Hz, *J* = 9.4 Hz 1 H), 6.84-6.90 (m, 2 H), 7.33-7.39 (m, 1 H), 7.42-7.48 (m, 4 H).
- ¹³C-NMR: (90.6 MHz, CDCl₃): δ = 114.8 (d, *J*_{CF} = 22.2 Hz, CH), 116.4 (d, *J*_{CF} = 7.7 Hz, CH), 116.6 (d, *J*_{CF} = 22.5 Hz, CH), 127.6 (s, CH), 128.7 (d, *J*_{CF} = 7.0 Hz, C_{q}), 128.9 (4xCH), 138.6 (d, *J*_{CF} = 1.7 Hz, Cq), 139.6 (d, *J*_{CF} = 2.3 Hz, C_{q}), 156.3 (d, *J*_{CF} = 235.7 Hz, C_{q}).

3',4'-Dichlor-5-fluorbiphenyl-2-amin

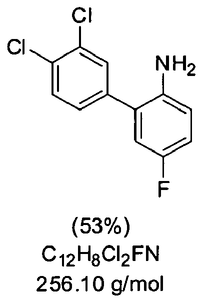

3',4'-Dichlor-5-fluorbiphenyl-2-amin wird aus 4-Fluoranilin (25.0 mmol, 2.40 mL) und 3,4-Dichlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Der Überschuss an 4-Fluoranilin wird durch Vakuumdestillation entfernt und das erhaltene Rohprodukt säulenchromatographisch (Kieselgel, Hexan / EtOAc = 5:1) aufgereinigt. Es wird 3',4'-Dichlor-5-fluorbiphenyl-2-amin (0.80 mmol, 205 mg, 53%) erhalten.
- *R*_{f}-Wert: 0.6 (Hexan / EtOAc = 5:1) [UV]
- ¹H-NMR: (360 MHz, CDCl₃): δ = 6.69 (dd, *J*_{HF} = 4.8 Hz, *J* = 8.8 Hz, 1 H), 6.82 (dd, *J* = 3.0 Hz, *J*_{HF} = 9.0 Hz, 1 H), 6.89 (ddd, *J* = 3.0 Hz, *J* = 8.1 Hz, *J*_{HF} = 8.8 Hz, 1 H), 7.29 (dd, *J* = 2.0 Hz, *J* = 8.2 Hz, 1 H), 7.52 (d, *J* = 8.2 Hz, 1 H), 7.56 *(*d, *J* = 2.1 Hz, 1 H).
- ¹³C-NMR: (90.6 MHz, CDCl₃): δ = 115.8 (d, *J*_{CF} = 22.3 Hz, CH), 116.4 (d, *J*_{CF} = 22.8 Hz, CH), 116.8 (d, *J*_{CF} = 7.7 Hz, CH), 125.9 (d, *J*_{CF} = 7.2 Hz, Cq), 128.3 (2xCH), 130.9 (2xCH), 131.8 (Cq), 133.1 (Cq), 138.5 (d, *J*_{CF} = 1.7 Hz, Cq), 139.5 (d, *J*_{CF} = 2.1 Hz, Cq), 156.3 (d, *J*_{CF} = 237.2 Hz, Cq).

5-Brom-4'-chlorbiphenyl-2-amin

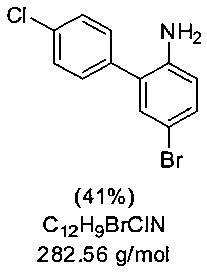

5-Brom-4'-chlorbiphenyl-2-amin wird aus 4-Bromanilin (20.0 mmol, 3.44 g) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Das erhaltene Rohprodukt wird mittels Säulenchromatographie (Kieselgel, Hexan / EtOAc = 6:1 → 4:1) gereinigt, wodurch 5-Brom-4'-chlorbiphenyl-2-amin (0.62 mmol, 175 mg, 41%) erhalten wird.
- *R*_{f}-Wert: 0.6 (Hexan / EtOAc = 4:1) [UV]
- ¹H-NMR: (600 MHz, CDCl₃): δ = 6.67 (d, *J =* 8.5 Hz, 1 H), 7.21 (d, *J* = 2.3 Hz, 1 H), 7.25 (dd, *J =* 2.3 Hz, *J =* 8.5 Hz, 1 H), 7.36 (d, *J=* 8.6 Hz, 2 H), 7.42 (d, *J* = 8.6 Hz, 2 H).
- ¹³C-NMR: (151 MHz, CDCl₃): δ = 117.4 (Cq), 129.2 (2xCH), 130.3 (2xCH), 130.6 (Cq), 131.4 (CH), 132.1 (Cq), 132.7 (CH), 133.7 (Cq), 136.4 (Cq). Ein CH-Signal fehlt aufgrund von Überlappung.

4'-Chlor-5-cyanobiphenyl-2-amin

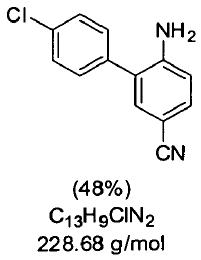

4'-Chlor-5-cyanobiphenyl-2-amin wird aus 4-Aminobenzonitril (20.0 mmol, 2.36 g) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Ethylacetat extrahiert. Das erhaltene Rohprodukt wird mittels Säulenchromatographie (Kieselgel, Hexan / EtOAc = 3:1 → 2:1) gereinigt, wodurch 4'-Chlor-5-cyanobiphenyl-2-amin (0.72 mmol, 165 mg, 48%) erhalten wird.
- *R*_{f}-Wert: 0.4 (Hexan / EtOAc = 2:1) [UV]
- ¹H-NMR: (600 MHz, CDCl₃): δ = 6.74 (d, *J*= 8.4 Hz, 1 H), 7.33-7.35 (m, 3 H), 7.42 (dd, *J*= 1.9 Hz, *J* = 8.4 Hz, 1 H), 7.45 (d, *J*= 8.3 Hz, 2 H).
- ¹³C-NMR: (151 MHz, CDCl₃): δ = 100.7 (Cq), 115.2 (CH), 119.8 (Cq), 126.1 (Cq), 129.5 (2xCH), 130.2 (2xCH), 132.9 (CH), 134.2 (Cq), 134.3 (CH), 135.5 (Cq), 147.5 (Cq).

4'-Chlor-5-ethoxybiphenyl-2-amin

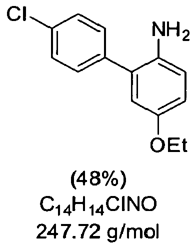

4'-Chlor-5-ethoxybiphenyl-2-amin wird aus p-Phenetidin (20.0 mmol, 2.59 g) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Das erhaltene Rohprodukt wird mittels Säulenchromatographie (Kieselgel, Hexan / EtOAc = 6:1 → 4:1) gereinigt, wodurch 4'-Chlor-5-ethoxybiphenyl-2-amin (0.72 mmol, 178 mg, 48%) erhalten wird.
- *R*_{f}-Wert: 0.3 (Hexan / EtOAc = 4:1) [UV]
- ¹H-NMR: (360 MHz, CDCl₃): δ = 1.38 (t, *J*= 7.0 Hz, 3 H), 3.98 (q, *J* = 7.0 Hz, 2 H), 6.67-6.78 (m, 3 H), 7.39 (s, 4 H).
- ¹³C-NMR: (90.6 MHz, CDCl₃): δ = 15.0 (CH₃), 64.2 (CH₂), 115.5 (CH), 116.6 (CH), 117.2 (CH), 127.6 (Cq), 128.9 (2xCH), 130.4 (2xCH), 133.2 (Cq), 136.7 (Cq), 137.9 (Cq), 152.2 (Cq).

5-Fluor-4'-methoxybiphenyl-2-amin

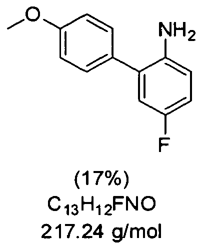

5-Fluor-4'-methoxybiphenyl-2-amin wird aus 4-Fluoranilin (20.0 mmol, 1.90 mL) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Der Überschuss an 4-Fluoranilin wird durch Vakuumdestillation entfernt und das erhaltene Rohprodukt säulenchromatographisch (Kieselgel, Hexan / EtOAc = 6: 1) gereinigt. Es wird 5-Fluor-4'-methoxybiphenyl-2-amin (0.26 mmol, 55 mg, 17%) erhalten.
- *R*_{f}-Wert: 0.2 (Hexan / EtOAc = 4:1) [UV]
- ¹H-NMR: (600 MHz, CDCl₃): δ = 3.84 (s, 3 H), 6.74 (dd, *J_{HF}* = 4.8 Hz, *J* = 9.1 Hz, 1 H), 6.83-6.86 (m, 2 H), 6.97 (d, *J* = 8.7 Hz, 2 H), 7.36 (d, *J*= 8.7 Hz, 2 H).
- ¹³C-NMR: (90.6 MHz, CDCl₃): δ = 55.3 (CH₃), 114.3 (2xCH), 114.5 (d, *J*_{CF} = 22.2 Hz, CH), 116.7 (d, *J*_{CF} = 22.3 Hz, CH), 116.9 (d, *J*_{CF} = 7.9 Hz, CH), 128.5 (d, *J*_{CF} = 7.3 Hz, Cq), 130.1 (2xCH), 130.6 (d, *J*_{CF} = 1.7 Hz, Cq), 138.6 (d, *J*_{CF} = 2.2 Hz, C_{q}), 156.7 (d, *J*_{CF} = 237.2 Hz, Cq), 159.1 (Cq).

5-Chlor-4'-fluorbiphenyl-2-amin

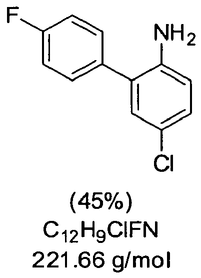

5-Chlor-4'-fluorbiphenyl-2-amin wird aus 4-Chloranilin (20.0 mmol, 2.54 g) und 4-Fluorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Das erhaltene Rohprodukt wird mittels Säulenchromatographie (Kieselgel, Hexan / EtOAc = 5:1) gereinigt, wodurch 5-Chlor-4'-fluorbiphenyl-2-amin (0.68 mmol, 151 mg, 45%) erhalten wird.
- *R*_{f}-Wert: 0.4 (Hexan / EtOAc = 4:1) [UV]
- ¹H-NMR: (360 MHz, CDCl₃): δ = 6.69 (d, J= 8.4 Hz, 1 H), 7.07 (d, J= 2.2 Hz, 1 H), 7.08-7.16 (m, 3 H), 7.39 (dd, *J* = 5.3 Hz, *J*= 8.8 Hz, 2 H).
- ¹³C-NMR: (90.6 MHz, CDCl₃): δ = 115.9 (d, *J*_{CF} = 21.4 Hz, 2xCH), 116.7 (CH), 123.2 (CH), 127.9 (Cq), 128.3 (CH), 130.0 (d, *J*_{CF} = 0.7 Hz, Cq), 130.7 (d, *J*_{CF} = 8.1 Hz, 2xCH), 134.2 (d, *J*_{CF} = 3.5 Hz, Cq), 142.2 (Cq), 162.3 (d, *J*_{CF} = 247.2 Hz, C_{q}).

2'-Brom-5-fluorbiphenyl-2-amin

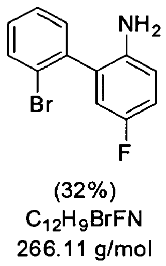

2'-Brom-5-fluorbiphenyl-2-amin wird aus 4-Fluoranilin (20.0 mmol, 1.90 mL) und 2-Bromphenyldiazoniumtetrafluoroborat (2.00 mmol, 0.54 g) analog der allgemeinen Arbeitsvorschrift AAV 3 synthetisiert. Es wird mit Diethylether extrahiert. Der Überschuss an 4-Fluoranilin wird durch Vakuumdestillation entfernt und das erhaltene Rohprodukt säulenchromatographisch (Kieselgel, Hexan / EtOAc = 10:1 → 4:1) gereinigt. Es wird 2'-Brom-5-fluorbiphenyl-2-amin (0.48 mmol, 128 mg, 32%) erhalten.
- *R*_{f}-Wert: 0.3 (Hexan / EtOAc = 4:1) [UV]
- ¹H-NMR: (600 MHz, CDCl₃): δ = 3.29 (s, 2 H), 6.70 (dd, J_{HF} = 4.8 Hz, *J* = 8.7 Hz, 1 H), 6.77 (dd, *J* = 3.0 Hz, *J*_{HF} = 8.9 Hz, 1 H), 6.92 (ddd, J = 3.0 Hz, *J*_{HF} = 8.2 Hz, J= 8.7 Hz, 1 H), 7.22-7.32 (m, 2 H), 7.35-7.41 (m, 1 H), 7.69 (dd, *J* = 1.2 Hz, *J* = 8.0 Hz, 1 H).
- ¹³C-NMR: (90.6 MHz, CDCl₃): δ = 115.6 (d, *J*_{CF} = 22.3 Hz, CH), 116.6 (d, *J*_{CF} = 22.7 Hz, CH), 116.5 (d, *J*_{CF} = 7.7 Hz, CH), 123.9 (Cq), 127.9 (CH), 128.0 (d, *J*_{CF} = 7.6 Hz, C_{q}), 129.6 (CH), 131.6 (CH), 133.2 (CH), 139.0 (d, *J*_{CF} = 1.6 Hz, Cq), 139.7 (d, *J*_{CF} = 2.1 Hz, Cq), 155.9 (d, *J*_{CF} = 236.8 Hz, Cq).

4'-Chlorbiphenyl-2-amin und 4'-Chlorbiphenyl-4-amin

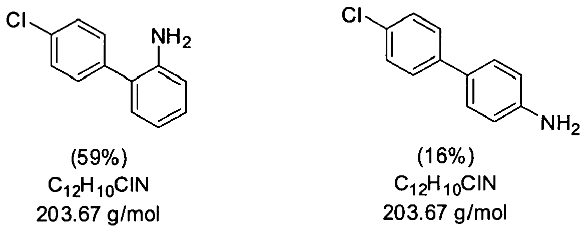

4'-Chlorbiphenyl-2-amin und 4'-Chlorbiphenyl-4-amin werden aus Anilin (20.0 mmol, 2.33 g) und 4-Chlorphenyldiazoniumchlorid (2.00 mmol, 5.00 mL der nach der allgemeinen Arbeitsvorschrift AAV 1 hergestellten 0.4 M Aryldiazoniumchloridlösung) analog der allgemeinen Arbeitsvorschrift AAV 3 synthetisiert. Es wird mit Diethylether extrahiert. Die beiden Regioisomere werden über Säulenchromatographie (Kieselgel, Hexan / EtOAc = 4:1) getrennt. Es wird 4'-Chlorbiphenyl-2-amin (0.88 mmol, 179 mg, 59%) und 4'-Chlorbiphenyl-4-amin (0.24 mmol, 49 mg, 16%) erhalten.

### 4'-Chlorbiphenyl-2-amin:

- *R*_{f}-Wert: 0.6 (Hexan / EtOAc = 4:1) [UV]
- ¹H-NMR: (360 MHz, CDCl₃): δ = 6.76 (dd, *J* = 0.9 Hz, *J*= 8.0 Hz, 1 H), 6.82 (dt, *J*= 1.1 Hz, *J*= 7.47 Hz, 1 H), 7.09 (dd, *J*= 1.4 Hz, *J*= 7.6 Hz, 1 H), 7.16 (ddd, *J* = 1.6 Hz, *J* = 7.4 Hz, *J* = 8.0 Hz, 1 H), 7.37-7.45 (m, 4 H).
- ¹³C-NMR: (90.6 MHz, CDCl₃): δ = 115.7 (CH), 118.8 (CH), 126.3 (Cq), 128.8 (CH), 129.0 (2xCH), 130.3 (CH), 130.4 (2xCH), 133.1 (Cq), 137.9 (Cq), 143.4 (C_{q}).

### 4'-Chlorbiphenyl-4-amin:

- *R*_{f}-Wert: 0.3 (Hexan / EtOAc = 4:1) [UV]
- ¹H-NMR: (600 MHz, CDCl₃): δ = 6.75 (d, *J*= 8.6 Hz, 2 H), 7.35 (d, *J* = 8.6 Hz, 2 H), 7.37 (d, *J*= 8.6 Hz, 2 H), 7.45 (d, *J=* 8.5 Hz, 2 H).
- ¹³C-NMR: (151 MHz, CDCl₃): δ = 115.4 (2xCH), 127.5 (2xCH), 127.8 (2xCH), 128.7 (2xCH), 130.2 (Cq), 132.1 (Cq), 139.6 (Cq), 146.1 (Cq).

## Patentansprüche

1. Verfahren zur Herstellung einer Verbindung der Formel 3 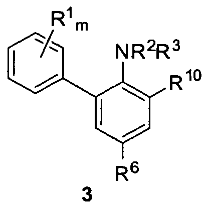 wobei eine Verbindung der Formel 1 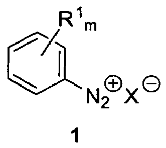 mit einer Verbindung der Formel 2 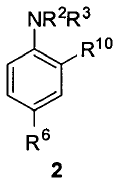 umgesetzt wird, wobei
m für 0, 1, 2, 3, 4 oder 5 steht;
jedes R¹ jeweils unabhängig für Halogen, Alkyl, Haloalkyl, Hydroxy, Hydroxyalkyl, Alkoxy, Haloalkoxy, Alkylthio, Cycloalkyl, Haloalkylthio, Alkenyl, Alkinyl, Amino, Nitro, Cyano, -SO₃R⁵, -SO₂NH₂, -SO₂NHR⁴, -SO₂NR⁴R⁵, -COOR⁴, -CONHR⁴, -CONR⁴R⁵, -COR⁴, -OCOR⁴, -NR⁴R⁵, -NR⁴COR⁵, -NR⁴SO₂R⁵, Alkylcarbonyl, Haloalkylcarbonyl, Alkenylcarbonyl, Alkoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Heteroarylalkyl, Arylalkoxycarbonyl, Arylalkylimino oder Heteroaryl steht;
X⁻ für Halogenid, Hydrogensulfat, Sulfat, Tetrafluoroborat, Acetat, Trifluoracetat, Hexafluorophosphat, Hexafluoroantimonat, das Anion eines aromatischen 1,2-Dicarbonsäureimids oder das Anion eines aromatischen 1,2-Disulfonsäureimids steht;
R² und R³ jeweils unabhängig für Wasserstoff, Alkyl, Hydroxyalkyl, Aminoalkyl, Cycloalkyl, Haloalkyl, -(CH₂)ₙ-OR⁴, -(CH₂)ₙ-NR⁴R⁵, -(CH₂)ₙ-NR⁴COR⁵, -(CH₂)ₙ-NR⁴COOR⁵, -(CH₂)ₙ-COOR⁴, -(CH₂)ₙ-CONHR⁴, -(CH₂)ₙ-CONR⁴R⁵, -(CH₂)ₙ-SO₃R⁴, -(CH₂)ₙ-CN, Arylalkyl, Heteroarylalkyl, Aryl oder Heteroaryl steht, oder ein R² und ein R³ zusammen einen Alkylidenrest bilden, oder ein R² und ein R³ zusammen ein nichtaromatisches Ringsystem bilden, welches 4, 5, 6, oder 7 Ringatome enthält, ausgewählt aus O, S, N und Kohlenstoffatomen, vorzugsweise mit Kohlenstoffatomen, oder ein R² und ein R¹⁰ zusammen ein nichtaromatisches Ringsystem bilden, welches 4, 5, 6, oder 7 Ringatome enthält, ausgewählt aus O, S, N und Kohlenstoffatomen, vorzugsweise mit Kohlenstoffatomen, oder ein R³ und ein R¹⁰ zusammen ein nichtaromatisches Ringsystem bilden, welches 4, 5, 6, oder 7 Ringatome enthält, ausgewählt aus O, S, N und Kohlenstoffatomen, vorzugsweise mit Kohlenstoffatomen;
n jeweils unabhängig für 1, 2, 3, 4, 5, 6, 7, 8, 9 oder 10 steht;
R⁴ jeweils unabhängig für Wasserstoff, Alkyl, Cycloalkyl, Haloalkyl, Arylalkyl, Heteroarylalkyl, Aryl oder Heteroaryl steht;
R⁵ jeweils unabhängig für Wasserstoff, Alkyl, Cycloalkyl, Haloalkyl, Arylalkyl, Heteroarylalkyl, Aryl oder Heteroaryl steht;
R⁶ jeweils unabhängig für Wasserstoff, Halogen, Alkyl, Alkenyl, Alkinyl, Cycloalkyl, Arylalkyl, Heteroarylalkyl, Haloalkyl, Hydroxyalkyl, Alkoxyalkyl, Aryloxyalkyl, Heteroaryloxyalkyl, Aminoalkyl, -(CH₂)ₙ-NR⁴R⁵, -COOH, -CHO, -CN, -COR⁴, Alkylcarbonyl, Haloalkylcarbonyl, Cycloalkylcarbonyl, Arylalkylcarbonyl, Alkenylcarbonyl, Arylcarbonyl, Heteroarylcarbonyl, -COOR⁴, Alkoxycarbonyl, Haloalkoxycarbonyl, Cycloalkoxycarbonyl, Arylalkoxycarbonyl, Alkenyloxycarbonyl, Aryloxycarbonyl, Heteroaryloxycarbonyl, -CONHR⁴, -CONR⁴R⁵, Amino, Nitro, -NHR⁴, -NR⁴R⁵, 1-Pyrrolidino, 1-Piperidino, 1-Morpholino, Alkylimino, Cycloalkylimino, Haloalkylimino, Arylalkylimino, -NR⁴COR⁵, -NR⁴COOR⁵, -NR⁴SO₂R⁵, Hydroxy, Alkoxy, Haloalkoxy, Cycloalkoxy, Arylalkyloxy, Aryloxy, Heteroaryloxy, -OCOR⁴, Alkylcarbonyloxy, Haloalkylcarbonyloxy, Cycloalkylcarbonyloxy, Arylalkylcarbonyloxy, Arylcarbonyloxy, Heteroarylcarbonyloxy, -OCONR⁴R⁵, -O-(CH₂)ₙ-OR⁴, -O-(CH²)ₙ-NR⁴R⁵, -O-(CH₂)ₙ-NR⁴COR⁵, -O-(CH₂)ₙ-NR⁴COOR⁵, -O-(CH₂)ₙ-COOR⁴, -O-(CH₂)ₙ-CONHR⁴, -O-(CH₂)ₙ-CONR⁴R⁵, -O-(CH₂)ₙ-SO₃R⁴, -O-(CH₂)ₙ-SO₂R⁴, -O-(CH₂)ₙ-CN, -SH, Alkylthio, Haloalkylthio, Cycloalkylthio, Arylalkylthio, Arylthio, Heteroarylthio, Alkylsulfonyl, Haloalkylsulfonyl, Cycloalkylsulfonyl, Arylalkylsulfonyl, Arylsulfonyl, Heteroarylsulfonyl, -SO₂NH₂, -SO₂NHR⁴, -SO₂NR⁴R⁵, -SO₃R⁵, Aryl oder Heteroaryl steht;
R¹⁰ jeweils unabhängig für Wasserstoff, Halogen, Alkyl, Haloalkyl, Hydroxyalkyl, Cycloalkyl, Arylalkyl, Heteroarylalkyl, -(CH₂)_{q}-NR⁴R⁵, -(CH₂)_{q}-NR⁴COR⁵, -(CH₂)_{q}-NR⁴COOR⁵, -(CH₂)_{q}-COOR⁴, -(CH₂)_{q}-CONHR⁴, -(CH₂)_{q}-CONR⁴R⁵, -(CH₂)_{q}-SO₃R⁴, -(CH₂)_{q}-CN, Aryl oder Heteroaryl steht;
q jeweils unabhängig für 1, 2, 3, 4, oder 5 steht,
**dadurch gekennzeichnet, dass** die Umsetzung im basischen Bereich durchgeführt wird.

2. Verfahren zur Herstellung einer Verbindung der Formel 3 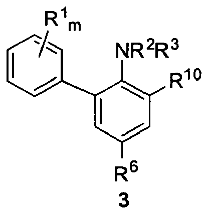 wobei in einem ersten Schritt eine Verbindung der Formel 1 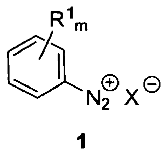 im basischen Bereich zu Verbindungen der Formeln **1a** und/oder **1b** und/oder **1c** umgesetzt wird, 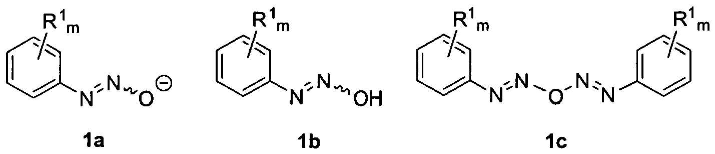 und in einem zweiten Schritt die Verbindungen 1a und/oder **1b** und/oder **1c** im basischen Bereich mit einer Verbindung der Formel 2 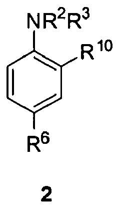 zu einer Verbindung der Formel 3 umgesetzt werden, 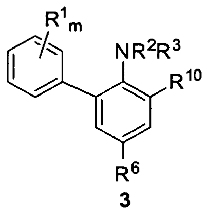 wobei R¹, R², R³, R⁶, R¹⁰, m und X- wie in Anspruch 1 definiert sind.

3. Verfahren nach einem der voranstehenden Ansprüche, **dadurch gekennzeichnet, dass** die Umsetzung bei einem pH von 9,1 oder größer durchgeführt wird.

4. Verfahren nach einem der voranstehenden Ansprüche **dadurch gekennzeichnet, dass** die Umsetzung in Gegenwart von mindestens einem Lösungsmittel, vorzugsweise Wasser, durchgeführt wird.

5. Verfahren nach einem der voranstehenden Ansprüche **dadurch gekennzeichnet, dass** die Umsetzung in Gegenwart von Wasser und mindestens einer Base durchgeführt wird.

6. Verfahren nach Anspruch 5, **dadurch gekennzeichnet, dass** die Base Natriumhydroxid oder Kaliumhydroxid ist.

7. Verfahren nach einem der voranstehenden Ansprüche **dadurch gekennzeichnet, dass** R¹ Fluor, Chlor, Brom oder Methoxy ist, R², R³ und R¹⁰ Wasserstoffatome sind, R⁶ Wasserstoff, Fluor, Chlor, Brom, CN, Methoxy oder Ethoxy ist und m gleich 0, 1, 2 oder 3 ist.

8. Verfahren nach einem der voranstehenden Ansprüche **dadurch gekennzeichnet, dass** die Umsetzung im Temperaturbereich von 50 bis 130°C, insbesondere von 70 °C bis 110°C durchgeführt wird.

9. Verfahren nach einem der voranstehenden Ansprüche **dadurch gekennzeichnet, dass** die Umsetzung in Gegenwart mindestens eines Reduktionsmittels durchgeführt wird.

10. Verfahren nach einem der voranstehenden Ansprüche **dadurch gekennzeichnet, dass** die Umsetzung unter elektrochemischer Reduktion durchgeführt wird.

11. Verfahren nach einem der voranstehenden Ansprüche **dadurch gekennzeichnet, dass** die Umsetzung unter Bestrahlung, Ultraschall oder Radiolyse durchgeführt wird.

12. Verfahren nach einem der voranstehenden Ansprüche **dadurch gekennzeichnet, dass** die Umsetzung unter Schutzgas durchgeführt wird.
